(19) Europäisches Patentamt / European Patent Office / Office européen des brevets

(11) **EP 4 755 394 A1**

(12) # EUROPEAN PATENT APPLICATION

published in accordance with Art. 153(4) EPC

(43) Date of publication:
**10.06.2026 Bulletin 2026/24**

(21) Application number: **24851157.8**

(22) Date of filing: **27.09.2024**

(51) International Patent Classification (IPC):
***A61K 38/17*** (2006.01) ***C07K 14/47*** (2006.01)
***C12N 15/12*** (2006.01) ***C12Q 1/6886*** (2018.01)
***C12N 15/85*** (2006.01) ***A61K 38/16*** (2006.01)
***A61P 35/00*** (2006.01) ***G01N 33/68*** (2006.01)
***G01N 33/574*** (2006.01)

(52) Cooperative Patent Classification (CPC):
**A61K 38/16; A61K 38/17; A61P 35/00; C07K 14/435; C07K 14/47; C12N 15/85; C12Q 1/6886; G01N 33/575; G01N 33/68**

(86) International application number:
**PCT/CN2024/121856**

(87) International publication number:
**WO 2025/031513 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.08.2023 CN 202310995091**
**08.08.2024 CN 202411084331**

(71) Applicant: **Nanjing Anji Biotechnology Co., Ltd.**
**Nanjing, Jiangsu 210000 (CN)**

(72) Inventors:
- **XU, Hanmei**
  **Nanjing, Jiangsu 210000 (CN)**
- **LI, Mengwei**
  **Nanjing, Jiangsu 210000 (CN)**
- **SARRA, Setrerrahmane**
  **Cheraga, Algiers16014 (DZ)**

(74) Representative: **Prüfer & Partner mbB**
**Patentanwälte · Rechtsanwälte**
**Sohnckestraße 12**
**81479 München (DE)**

Remarks:
•The filing date of the international application is within two months from the date of expiration of the priority period (R. 26bis.3 PCT)
•The complete document including Reference Table(s) and the Sequence Listing(s) can be downloaded from the EPO website

(54) **NOVEL POLYPEPTIDE, POLYPEPTIDE DERIVATIVE AND USE THEREOF**

(57) Disclosed are a novel polypeptide, a polypeptide derivative and the use thereof, which belong to the technical field of biomedicine. The present application specifically relates to a specific sequence, a discovery process, specific types of tumors to be resisted, a long-acting modification method and the use of the novel polypeptide, wherein the tumors include one or more of glioma, neuroblastoma, head and neck cancer, esophageal cancer, thyroid cancer, lung cancer, liver cancer, kidney cancer, breast cancer, cervical cancer, uterine cancer, ovarian cancer, colon cancer, small intestine cancer, ileocecal cancer, gastric cancer, bladder cancer, pancreatic cancer, prostate cancer, cholangiocarcinoma, melanoma, sarcoma, myeloma, lymphoma and leukemia; and the specific use comprises the inhibition of the proliferation and/or metastasis of the above-mentioned tumor cells. The novel polypeptide has a wide therapeutic spectrum and important therapeutic value against various tumors.

EP 4 755 394 A1

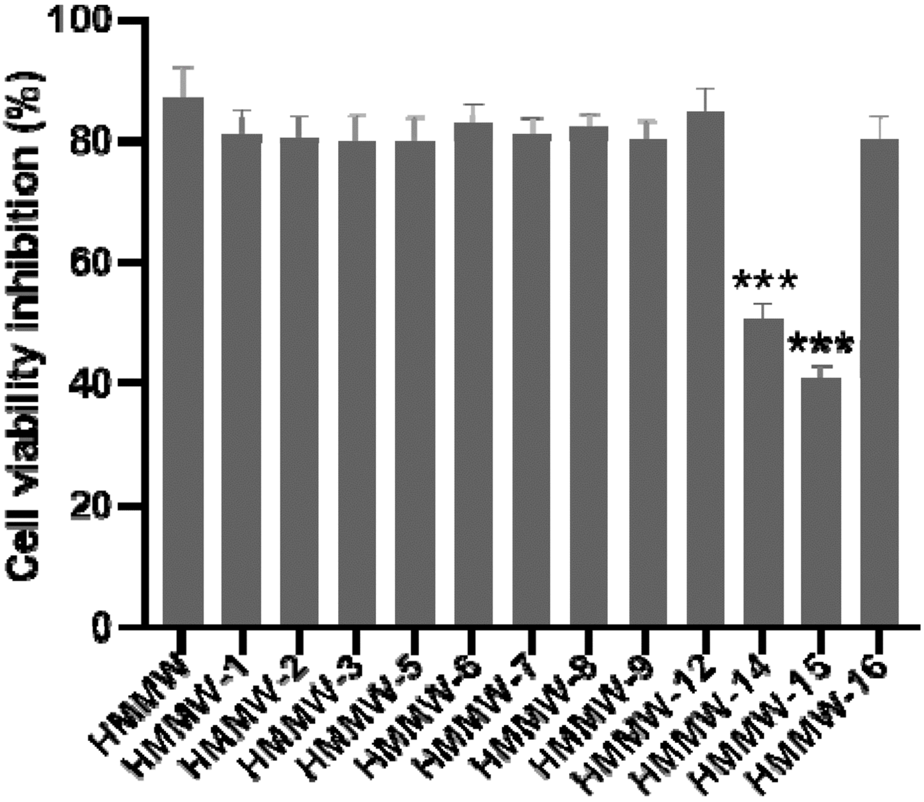
Cell viability inhibition (%)
100
80
60
40
20
0
***
***
HMMW
HMMW-1
HMMW-2
HMMW-3
HMMW-5
HMMW-6
HMMW-7
HMMW-8
HMMW-9
HMMW-12
HMMW-14
HMMW-15
HMMW-16

FIG. 1

## Description

### TECHNICAL FIELD

The present application belongs to the technical field of biomedicine, specifically relates to a novel polypeptide, a polypeptide derivative and uses thereof, and more specifically relates to a novel polypeptide, a polypeptide derivative and uses thereof in tumor prevention or treatment.

### BACKGROUND

Open reading frames (ORFs) for encoding proteins are composed of a string of sense codons, starting from a start codon and ending at a stop codon. Due to the limitations of traditional computer algorithms and detection methods, most algorithms for predicting ORFs have a minimum lower limit of 100 amino acids. With the development of ribosome profiling sequencing technology, computational biology, and high-throughput omics technology, an increasing number of studies have shown that many non-coding RNAs actually contain small open reading frames (sORFs) with a length of less than 300 nt, which can encode functional polypeptides with a length of less than 100 amino acids, known as micropeptides. Transcript sources of micropeptides mainly include non-coding RNAs (lncRNAs, circRNAs, pri-miRNAs), ribosomal RNAs (rRNA), antisense transcripts, 5'UTR, 3'UTR and intergenic ORFs.

The earliest research on micropeptides began in 1996, when German scientists discovered a 679 nt RNA transcribed from an ENOD40 (early nodulin 40) gene in leguminous plants, and the RNA exerts non-coding functions. Based on the length greater than 200 nt, the RNA was classified as a lncRNA at the time and can actually encode proteins and regulate plant growth. The specific results were published in the journal Science. In 2002, German researchers conducted a more detailed study of the RNA and found that the RNA contained two sORFs which indeed encoded micropeptides (one encoding a 12-amino acid polypeptide and the other encoding a 24-amino acid polypeptide), and confirmed the ability of the RNA to interact with a sucrose synthase. In the following 20 years, with the development of computational biology and multi-omics deep sequencing technologies (e.g., ribosomal imprinting sequencing and polypeptide omics sequencing), scientists have discovered a large number of previously unannotated sORFs hidden in non-coding RNAs in a plurality of species such as nematodes, fruit flies, zebrafish, plants, mice, and humans. These sORFs can translate completely new polypeptides involved in biological functions such as muscle development, mRNA modification, immune regulation and tumor development, opening up a new perspective for proteomics research.

In recent years, some studies have reported that micropeptides (e.g., SMIM22, HOXB-AS3, SMIM30, MP31, and PINT87aa) encoded by ncRNAs can regulate occurrence and development of different tumors (including breast cancer, colorectal cancer, glioma, liver cancer, melanoma and esophageal squamous cell carcinoma), and can specifically play a role in promoting or inhibiting proliferation, metastasis, energy metabolism, drug resistance, and the like of tumors, suggesting that micropeptides can be in-depth developed as potential tumor diagnostic markers or therapeutic targets.

The Chinese invention patent with the publication number CN112442116A records a micropeptide HMMW encoded by a lncRNA and a use thereof in detection and treatment of malignant tumors such as head and neck cancer, thyroid cancer, and kidney cancer. HMMW is a completely new endogenous polypeptide sequence, specifically consisting of 51 amino acid residues, and a direct binding receptor is an AQP2 protein.

### SUMMARY

1. Objectives of the invention

Regarding the new micropeptide HMMW first identified by the applicant (refer to the applicant's previous patent with a granted publication number CN112442116B for details),

a first objective of the present application is to verify the function of polypeptides that have homology with the micropeptide HMMW;

a second objective of the present application is to study key active amino acids of the micropeptide HMMW and provide a homologous functional fragment of the micropeptide HMMW that is shorter in length but has the same or substantially the same function, namely the novel polypeptide in the present application;

a third objective of the present application is to provide a derivative of the novel polypeptide, such as a derivative of the novel polypeptide obtained by long-acting modification, to better preserve the in vivo activity of the novel polypeptide, improve stability, and prolong the half-life; and

a fourth objective of the present application is to further provide uses of the novel polypeptide or the derivative thereof in preparation of tumor prevention or treatment drugs, and specifically the uses comprise prevention or treatment effects on human glioma, neuroblastoma, head and neck cancer, esophageal cancer, thyroid cancer, lung cancer,

liver cancer, kidney cancer, breast cancer, cervical cancer, uterine cancer, ovarian cancer, colon cancer, small intestine cancer, ileocecal cancer, gastric cancer, bladder cancer, pancreatic cancer, prostate cancer, cholangiocarcinoma, melanoma, sarcoma, myeloma, lymphoma, leukemia and the like.

2. Technical solution

To achieve the objectives of the invention, the technical solution adopted in the present application is as follows: The present application provides a novel polypeptide, and the novel polypeptide is based on a completely new micropeptide HMMW (SEQ ID NO.1) discovered by the inventor, has been determined to have the same or substantially the same function as the micropeptide HMMW by means of sequence modification, structural analysis, amino acid mutation and the like, and is specifically selected from any one of the following:

(1): a polypeptide with the same or substantially the same function formed by deleting 3 consecutive amino acids from a polypeptide having the amino acid sequence set forth inset forth in SEQ ID NO.1;
(2): a polypeptide with the same or substantially the same function formed by deleting 1to 34 amino acids at N-terminus of a polypeptide having the amino acid sequence set forth in SEQ ID NO.1; and
(3): a polypeptide with the same or substantially the same function formed by substituting one or more amino acids of a polypeptide having the amino acid sequence set forth in SEQ ID NO.1, or (1), or (2).

Further, the novel polypeptide is a polypeptide with the same or substantially the same function formed by deleting 3 consecutive amino acids from a polypeptide having the amino acid sequence set forth in SEQ ID NO.1.
Further, the novel polypeptide is a polypeptide with the same or substantially the same function formed by deleting 3 consecutive amino acids at positions 3N-2, 3N-1, and 3N from a polypeptide having the amino acid sequence set forth in SEQ ID NO.1, wherein N=1, 2, 3, 4, 5, 6, 7, 8, 9, 10, 11, 12, 13, 14, 15, 16, or 17.
Further, the amino acid sequence of the novel polypeptide is shown as any one of SEQ ID NO.2 to SEQ ID NO.18.
Further, the novel polypeptide is a polypeptide with the same or substantially the same function formed by deleting 3 consecutive amino acids at positions 3N-2, 3N-1, and 3N from a polypeptide having the amino acid sequence set forth in SEQ ID NO.1, wherein N=1, 2, 3, 5, 6, 7, 8, 9, 12, 14, 15, or 16.
Further, the amino acid sequence of the novel polypeptide is shown as any one of SEQ ID NO.2 to SEQ ID NO.4, SEQ ID NO.6 to SEQ ID NO.10, SEQ ID NO.13, or SEQ ID NO.15 to SEQ ID NO.17.
Further, the novel polypeptide is a polypeptide with the same or substantially the same function formed by deleting 3 consecutive amino acids at positions 3N-2, 3N-1, and 3N from a polypeptide having the amino acid sequence set forth in SEQ ID NO.1, wherein N=1, 2, 3, 5, 6, 7, 8, 9, 12, or 16.
Further, the amino acid sequence of the novel polypeptide is shown as any one of SEQ ID NO.2 to SEQ ID NO.4, SEQ ID NO.6 to SEQ ID NO.10, SEQ ID NO.13, or SEQ ID NO.17.
Further, the novel polypeptide is a polypeptide with the same or substantially the same function formed by deleting 1-34 amino acids at N-terminus of the polypeptide having the amino acid sequence set forth in SEQ ID NO.1.
Further, the novel polypeptide is a polypeptide with the same or substantially the same function formed by deleting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or 34 amino acids at N-terminus of a polypeptide having the amino acid sequence set forth in SEQ ID NO.1.
Further, the novel polypeptide is a polypeptide with the same or substantially the same function formed by deleting 4, 9, 14, 19, 24, 29, or 34 amino acids at N-terminus of a polypeptide having the amino acid sequence set forth in SEQ ID NO.1.
Further, the amino acid sequence of the novel polypeptide is shown as any one of SEQ ID NO.19 to SEQ ID NO.25.
Further, the novel polypeptide is a polypeptide with the same or substantially the same function formed by deleting 1-19 amino acids at N-terminus of a polypeptide having the amino acid sequence set forth in SEQ ID NO.1.
Further, the novel polypeptide is a polypeptide with the same or substantially the same function formed by deleting 1, 2, 3, 4, 5, 6, 7, 8, 9, 10, 11, 12, 13, 14, 15, 16, 17, 18, or 19 amino acids at N-terminus of a polypeptide having the amino acid sequence set forth in SEQ ID NO.1.
Further, the novel polypeptide is a polypeptide with the same or substantially the same function formed by deleting 4, 9, 14, or 19 amino acids at N-terminus of a polypeptide having the amino acid sequence set forth in SEQ ID NO.1.
Further, the amino acid sequence of the novel polypeptide is shown as any one of SEQ ID NO.19 to SEQ ID NO.22.
Further, the novel polypeptide is a polypeptide with the same or substantially the same function formed by deleting 1-14 amino acids at N-terminus of a polypeptide having the amino acid sequence set forth in SEQ ID NO.1.
Further, the novel polypeptide is a polypeptide with the same or substantially the same function formed by

deleting 1, 2, 3, 4, 5, 6, 7, 8, 9, 10, 11, 12, 13, or 14 amino acids at N-terminus of a polypeptide having the amino acid sequence set forth in SEQ ID NO.1.

Further, the novel polypeptide is a polypeptide with the same or substantially the same function formed by deleting 4, 9, or 14 amino acids at N-terminus of a polypeptide having the amino acid sequence set forth in SEQ ID NO.1.

Further, the amino acid sequence of the novel polypeptide is shown as any one of SEQ ID NO.19 to SEQ ID NO.21.

Further, the novel polypeptide is a polypeptide with the same or substantially the same function formed by substituting one or more amino acids of a polypeptide having the amino acid sequence set forth in SEQ ID NO.1.

Further, the novel polypeptide is a polypeptide with the same or substantially the same function formed by substituting one amino acid of a polypeptide having the amino acid sequence set forth in SEQ ID NO.1.

Further, the substituting one amino acid refers to substituting the amino acid at the position 2, 3, 8, 9, 10, 11, 12, 13, 17, 19, 20, 21, 22, 25, 27, 28, 30, 31, 32, 40, 41, 42, 43, 44, 45, 48, 49, or 50 in the amino acid sequence set forth in SEQ ID NO.1, to form the polypeptide with the same or substantially the same function.

Further, the amino acid sequence of the novel polypeptide is shown as any one of SEQ ID NO.26 to SEQ ID NO.53.

Further, the substituting one amino acid refers to substituting the amino acid at the position 2, 3, 8, 9, 10, 11, 12, 13, 17, 19, 20, 21, 22, 25, 27, 28, 30, 31, 32, 40, 41, 42, 43, 44, 45, 48, or 50 in the amino acid sequence set forth in SEQ ID NO.1, to form the polypeptide with the same or substantially the same function.

Further, the amino acid sequence of the novel polypeptide is shown as any one of SEQ ID NO.26 to SEQ ID NO.51, and SEQ ID NO.53.

Further, the substituting one amino acid refers to substituting the amino acid at the position 2, 3, 8, 9, 10, 11, 12, 13, 17, 19, 20, 21, 22, 25, 27, 28, 30, 31, 32, 40, 41, 42, 44, 45, or 50 in the amino acid sequence set forth in SEQ ID NO.1, to form the polypeptide with the same or substantially the same function.

Further, the amino acid sequence of the novel polypeptide is shown as any one of SEQ ID NO.26 to SEQ ID NO.47, SEQ ID NO.49 to SEQ ID NO.50, and SEQ ID NO.53.

The present application further provides a nucleotide, and the nucleotide encodes one of the novel polypeptide.

The present application further provides a recombinant vector, and the recombinant vector comprises the nucleotide sequence for encoding the novel polypeptide, and may be a plasmid or viral vector or any other vector known to those skilled in the art.

The present application further provides a derivative of the novel polypeptide, and the derivative is a derivative with the same or substantially the same function of the novel polypeptide obtained by modification.

Further, the modification comprises one or more of N-terminus modification, C-terminus modification, side-chain modification, amino acid modification, or backbone modification.

Further, the modification comprises one or more long-acting modifications of polyethylene glycol modification, fatty acid linkage, fusion with Fc (igg1, igg4) polypeptide, binding with human serum albumin, non-natural amino acid substitution, polypeptide cyclization, or high-end formulations .

Furthermore, the polyethylene glycol modification mainly modifies an N-terminus, a C-terminus, a Lys side chain, and a thiol group of Cys of a polypeptide.

Further, the molecular weight range of single molecules of polyethylene glycol used for the polyethylene glycol modification is PEG5 to PEG40.

Further, the polyethylene glycol modification may use a single polyethylene glycol molecule or a plurality of polyethylene glycol molecules.

Further, the fatty acid linkage exerts a binding effect with serum albumin in vivo, and the linkage comprises direct N-terminus linkage and linkage with an 18-carbon fatty acid and polypeptide using a linker, with a small peptide Ala-Ala-Asn as the linker.

Further, the fatty acid linkage comprises N-terminus linkage.

Further, the N-terminus linkage comprises N-terminus linkage with a 12- to 20-carbon fatty acid. Still further, the N-terminus linkage comprises N-terminus linkage with an 18-carbon fatty acid.

Further, the fatty acid linkage comprises linkage with a fatty acid using a linker.

Further, the linkage using a linker comprises linkage with a 12- to 20-carbon fatty acid using a linker. Still further, the linkage using a linker comprises linkage with an 18-carbon fatty acid using a linker.

Further, the linker is a peptide less than 5 amino acids in length.

Further, the linker is a peptide less than 3 amino acids in length. Still further, the linker is the peptide Ala-Ala-Asn.

The present application further provides uses of the novel polypeptide or the derivative thereof, the nucleotide for encoding the novel polypeptide, or the recombinant vector in preparation of tumor prevention or treatment drugs.

The present application further provides uses of the polypeptide and pharmaceutically acceptable salts and esters thereof, or the polypeptide derivative and pharmaceutically acceptable salts and esters thereof in preparation of tumor prevention or treatment drugs.

Further, the tumors comprise one or more of glioma, neuroblastoma, head and neck cancer, esophageal cancer, thyroid cancer, lung cancer, liver cancer, kidney cancer, breast cancer, cervical cancer, uterine cancer, ovarian cancer, colon cancer, small intestine cancer, ileocecal cancer, gastric cancer, bladder cancer, pancreatic cancer, prostate cancer, cholangiocarcinoma, melanoma, sarcoma, myeloma, lymphoma, and leukemia.
Further, the tumor prevention or treatment comprises inhibiting proliferation and/or metastasis of tumor cells.
Further, the tumor prevention or treatment comprises inhibiting proliferation of tumor cells.
Further, the tumor prevention or treatment comprises inhibiting metastasis of tumor cells.
Further, the tumor cells in the inhibiting proliferation of tumor cells comprise:

cancer cells of the glioblastoma, comprising: U87-MG;
cancer cells of the neuroblastoma, comprising: SH-SY5Y;
cancer cells of the head and neck cancer, comprising: any one of oral epidermoid carcinoma cell KB, laryngeal epidermoid carcinoma cell HEp2, tongue squamous cell carcinoma cell CAL27, and nasopharyngeal carcinoma cell CNE-2Z;
cancer cells of the esophageal cancer, comprising: Eca-109 and/or TE-13;
cancer cells of the thyroid cancer, comprising: NPPA87-1;
cancer cells of the lung cancer, comprising: any one or combination of 95-D, SK-MES-1, SPC-A1, and A549;
cancer cells of the liver cancer, comprising: HCCLM3 and/or HepG2;
cancer cells of the kidney cancer, comprising: Ketr-3 and/or 786-O;
cancer cells of the breast cancer, comprising: SK-BR-3 and/or MDA-MB-231;
cancer cells of the cervical cancer, comprising: CaSki and/or Hela;
cancer cells of the uterine cancer, comprising: RL-952 and/or MS751;
cancer cells of the ovarian cancer, comprising: ES-2 and/or SKOV3;
cancer cells of the colon cancer, comprising: HT29 and/or SW480;
cancer cells of the small intestine cancer, comprising: HIC;
cancer cells of the ileocecal cancer, comprising: HCT-8;
cancer cells of the gastric cancer, comprising: MGC-803 and/or BGC-823;
cancer cells of the bladder cancer, comprising: T24 and/or EJ;
cancer cells of the pancreatic cancer, comprising: ASPC-1 and/or PANC-1;
cancer cells of the prostate cancer, comprising: 22RV1 and/or PC-3;
cancer cells of the cholangiocarcinoma, comprising: QBC939;
cancer cells of the melanoma, comprising: SK-mel-2 and/or A375;
cancer cells of the sarcoma, comprising: MG63 and/or HT-1080;
cancer cells of the myeloma, comprising: LP-1;
cancer cells of the lymphoma, comprising: any one or combination of Raji, RPMI 8226, U937, and WSU-DLCL2; and
cancer cells of the leukemia, comprising: any one or combination of CEM/C1, NB4, KG-1, K562, HL60, Jurkat, and Clone E6-1.

Further, tumor cells in the inhibiting metastasis of tumor cells comprise:

cancer cells of the melanoma, comprising: A375;
cancer cells of the cervical cancer, comprising: Hela;
cancer cells of the ovarian cancer, comprising: Skvo3;
cancer cells of the liver cancer, comprising: Hep3B; and
cancer cells of the lung cancer, comprising: A549.

The present application further provides a pharmaceutical composition for preventing or treating tumors, and the pharmaceutical composition comprises at least the novel polypeptide or the derivative thereof and a pharmaceutically acceptable carrier. The tumors comprise one or more of glioma, neuroblastoma, head and neck cancer, esophageal cancer, thyroid cancer, lung cancer, liver cancer, kidney cancer, breast cancer, cervical cancer, uterine cancer, ovarian cancer, colon cancer, small intestine cancer, ileocecal cancer, gastric cancer, bladder cancer, pancreatic cancer, prostate cancer, cholangiocarcinoma, melanoma, sarcoma, myeloma, lymphoma, and leukemia.

3. Beneficial effects

Compared with the related art, the beneficial effects of the present application are as follows:

(1) The novel polypeptide provided in the present application is a polypeptide obtained by performing deletion or

truncation on the micropeptide HMMW, consists of 17-50, especially 37-50 amino acids, and is shorter in length than the micropeptide HMMW. On the one hand, the shorter polypeptide has a lower molecular weight and is more conducive to absorption. As in Examples, at the same dose, the novel polypeptide HMMW15-51 has a significantly better inhibitory effects on proliferation of cells of human glioma, neuroblastoma, head and neck cancer, esophageal cancer, thyroid cancer, lung cancer, liver cancer, kidney cancer, breast cancer, cervical cancer, uterine cancer, ovarian cancer, colon cancer, small intestine cancer, ileocecal cancer, gastric cancer, bladder cancer, pancreatic cancer, prostate cancer, cholangiocarcinoma, melanoma, sarcoma, myeloma, lymphoma, and leukemia than those of the micropeptide HMMW, a significantly better inhibitory effect on metastasis of human melanoma cells than that of the micropeptide HMMW, and a comparable inhibitory effect on metastasis of cells of human ovarian cancer, liver cancer, and lung cancer to that of the micropeptide HMMW. On the other hand, a synthesis process can be simplified and synthesis costs are saved.

(2) The novel polypeptide provided in the present application is formed by substituting amino acids of the micropeptide HMMW or the polypeptide obtained by performing deletion or truncation on the micropeptide HMMW. The present application confirms that L43, R48, and R49 are key active sites in the micropeptide HMMW sequence by attempting to combine different screening methods and activity evaluation systems, providing a basis for secondary development of the micropeptide HMMW.

(3) The derivative of the novel polypeptide provided in the present application, comprising polyethylene glycol modification, fatty acid modification, or any other long-acting modification, can better preserve in vivo activity of the novel polypeptide, improve stability, and prolong the half-life. The present application determines long-acting modification schemes through experimentation and in vivo activity evaluation. The half-life and in vivo anti-tumor time of the novel polypeptide are significantly prolonged, and the novel polypeptide has potential applications in development of tumor drugs. At the same dose, the in vivo anti-tumor effect of the novel polypeptide obtained by long-acting modification is significantly better than that of the novel polypeptide, and the frequency of administration is significantly shortened.

## BRIEF DESCRIPTION OF THE DRAWINGS

FIG. 1 shows the effects of different deletants of a micropeptide HMMW on proliferation of tongue squamous cell carcinoma CAL27 cells;

FIG. 2 shows the effects of different truncated fragments of the micropeptide HMMW on proliferation of tongue squamous cell carcinoma CAL27 cells;

FIG. 3 shows sequence information of alanine scan mutations in the micropeptide HMMW;

FIG. 4 shows the effects of single amino acid mutation sequences of the micropeptide HMMW on proliferation of tongue squamous cell carcinoma CAL27 cells;

FIG. 5 shows statistical results of metastatic activity of melanoma A375 cells inhibited with the novel polypeptide and the micropeptide HMMW;

FIG. 6 shows statistical results of metastatic activity of cervical cancer Hela cells inhibited with the novel polypeptide and the micropeptide HMMW;

FIG. 7 shows statistical results of metastatic activity of ovarian cancer Skvo3 cells inhibited with the novel polypeptide and the micropeptide HMMW;

FIG. 8 shows statistical results of metastatic activity of liver cancer Hep3B cells inhibited with the novel polypeptide and the micropeptide HMMW;

FIG. 9 shows statistical results of metastatic activity of lung cancer A549 cells inhibited with the novel polypeptide and the micropeptide HMMW;

FIG. 10 shows inhibition results of the novel polypeptide and the micropeptide HMMW on the in vivo tumorigenicity of human tongue squamous cell carcinoma CAL27 cells;

FIG. 11 shows the effects of the novel polypeptide and a peptide obtained by long-acting modification on proliferation of human tongue squamous cell carcinoma CAL27 cells;

FIG. 12 shows the effects of the novel polypeptide and the peptide obtained by long-acting modification on proliferation of human ovarian cancer SKOV3 cells;

FIG. 13 shows the effects of the novel polypeptide and the peptide obtained by long-acting modification on proliferation of human liver cancer Hep3B cells; and

FIG. 14 shows inhibition results of the novel polypeptide and the peptide obtained by long-acting modification on the in vivo tumorigenicity of human tongue squamous cell carcinoma CAL27 cells.

## DETAILED DESCRIPTION

The present application is further described below with reference to specific examples.

## Example 1

This example provides the effects of different deletion mutants of a micropeptide HMMW on proliferation of human tongue squamous cell carcinoma CAL27 cells.

Due to the fact that the micropeptide HMMW (SEQ ID NO.1) is a completely new sequence, further development of smaller functional fragments thereof may reduce the cost of later development. Firstly, three amino acids in a group were sequentially deleted from an N-terminus (1-51aa) of the polypeptide, resulting in 17 different deletant polypeptides, respectively named HMMW-1 (1-3aa deleted, SEQ ID NO.2), HMMW-2 (4-6aa deleted, SEQ ID NO.3), HMMW-3 (7-9aa deleted, SEQ ID NO.4), HMMW-4 (10-12aa deleted, SEQ ID NO.5), HMMW-5 (13-15aa deleted, SEQ ID NO.6), HMMW-6 (16-18aa deleted, SEQ ID NO.7), HMMW-7 (19-21aa deleted, SEQ ID NO.8), HMMW-8 (22-24aa deleted, SEQ ID NO.9), HMMW-9 (25-27aa deleted, SEQ ID NO.10), HMMW-10 (28-30aa deleted, SEQ ID NO.11), HMMW-11 (31-33aa deleted, SEQ ID NO.12), HMMW-12 (34-36aa deleted, SEQ ID NO.13), HMMW-13 (37-39aa deleted, SEQ ID NO.14), HMMW-14 (40-42aa deleted, SEQ ID NO.15), HMMW-15 (43-45aa deleted, SEQ ID NO.16), HMMW-16 (46-48aa deleted, SEQ ID NO.17), HMMW-17 (49-51aa deleted, SEQ ID NO.18). In the deletant polypeptides obtained by chemical solid-phase synthesis, the five polypeptides HMMW-4, HMMW-10, HMMW-11, HMMW-13, and HMMW-17 may have undergone changes in amino acid composition and have poor solubility, and thus cannot conduct subsequent activity experiments for evaluation. Therefore, the other 12 deletant polypeptides with good solubility were selected to compare the effects thereof on proliferation of human tongue squamous cell carcinoma CAL27 cells.

When human tongue squamous cell carcinoma CAL27 cells were cultured to a density of 90% in a 37°C, 5% $CO_2$ incubator, the cells were digested with trypsin and collected, resuspended in a culture solution, and counted under a microscope. The cell concentration was adjusted to approximately $5\times10^4$ cells/mL, and a cell suspension was seeded into a 96-well plate with 100 μL per well, and cultured overnight in a 37°C, 5% $CO_2$ incubator. After the cells fully adhered to the wall, 100 μL of the 12 deletant polypeptides and HMMW having a concentration of 80 μM were added separately, and a culture solution without any drugs was used as a negative control group. After incubation was continued for 48 h, add 100 μL of CCK8 solution to each well of the 96-well plate. An absorbance OD was measured at a detection wavelength of 450 nm using a microplate reader, and a proliferation inhibition (PI) was calculated using a formula: PI(%)=1-Treatment group/Negative control group. The experiment was independently repeated 3 times, and the results were expressed as Mean±SD. A statistical T-test was performed, and the results are shown in Table 1 and FIG. 1.

Table 1 Effects of different deletion mutants of micropeptide HMMW on proliferation of human tongue squamous cell carcinoma CAL27 cells

| Group | 48 h | | Solubility |
|---|---|---|---|
| | OD | Proliferation inhibition (%) | PBS/0.1% DMSO |
| Negative control group | 2.048±0.241 | - | |
| HMMW | 0.260±0.098 | 87.302±4.785 | Soluble |
| HMMW-1 | 0.390±0.087 | 80.965±4.248 | Soluble |
| HMMW-2 | 0.404±0.076 | 80.251±3.711 | Soluble |
| HMMW-3 | 0.415±0.091 | 79.742±4.443 | Soluble |
| HMMW-5 | 0.410±0.079 | 79.992±3.857 | Soluble |
| HMMW-6 | 0.352±0.068 | 82.830±3.320 | Soluble |
| HMMW-7 | 0.392±0.059 | 80.840±2.881 | Soluble |
| HMMW-8 | 0.365±0.047 | 82.201±2.295 | Soluble |
| HMMW-9 | 0.405±0.061 | 80.234±2.979 | Soluble |
| HMMW-12 | 0.307±0.073 | 85.016±3.564 | Soluble |
| **HMMW-14** | 1.007±0.052 | **50.816±2.539*** ** | Soluble |
| **HMMW-15** | 1.208±0.038 | **41.022±1.855*** ** | Soluble |

(continued)

| Group | 48 h | | Solubility |
|---|---|---|---|
| | OD | Proliferation inhibition (%) | PBS/0.1% DMSO |
| HMMW-16 | 0.403±0.075 | 80.314±3.662 | Soluble |
| Note: Compared with the HMMW treatment group, * represents *P*<0.05, ** represents *P*<0.01, and *** represents *P*<0.001. | | | |

According to Table 1 and FIG. 1, in case of a normal solubility, compared with the HMMW treatment group, at the same dose, 10 deletant polypeptides HMMW-1, HMMW-2, HMMW-3, HMMW-5, HMMW-6, HMMW-7, HMMW-8, HMMW-9, HMMW-12, and HMMW-16 have comparable inhibitory effects to that of the HMMW on proliferation of the human tongue squamous cell carcinoma CAL27 cells, and do not have significantly decreased activity; and HMMW-14 and HMMW-15 have significantly decreased inhibitory effects on proliferation of the human tongue squamous cell carcinoma CAL27 cells compared to the HMMW, but still have activity.

**Example 2**

This example provides the effects of different truncated fragments of the micropeptide HMMW on proliferation of human tongue squamous cell carcinoma CAL27 cells.

Based on the results of Example 1, truncation was performed from the N-terminus of the micropeptide HMMW. Since HMMW-1 in Example 1 was obtained by deletion of the amino acid at the position 1-3aa, and it was found that an anti-tumor effect of the polypeptide was not affected, truncation was performed from the N-terminus, the amino acids at the positions 5-51, 10-51, 15-51, 20-51, 25-51, 30-51, and 35-51 were retained respectively, and the resulting polypeptides were named HMMW5-51 (SEQ ID NO.19), HMMW10-51 (SEQ ID NO.20), HMMW15-51 (SEQ ID NO.21), HMMW20-51 (SEQ ID NO.22), HMMW25-51 (SEQ ID NO.23), HMMW30-51 (SEQ ID NO.24), and HMMW35-51(SEQ ID NO.25) respectively. In the seven truncated polypeptides obtained by chemical solid-phase synthesis, the three polypeptides HMMW25-51, HMMW30-51, and HMMW35-51 may have undergone significant changes in amino acid composition and have poor solubility, and thus cannot conduct subsequent activity experiments for evaluation. Therefore, the four truncated polypeptides HMMW5-51, HMMW10-51, HMMW15-51, and HMMW20-51 with good solubility were selected to compare the effects thereof on proliferation of the human tongue squamous cell carcinoma CAL27 cells.

Similarly, when human tongue squamous cell carcinoma CAL27 cells were cultured to a density of 90% in a 37°C, 5% $CO_2$ incubator, the cells were digested with trypsin and collected, resuspended in a culture solution, and counted under a microscope. The cell concentration was adjusted to approximately $5\times10^4$ cells/mL, and a cell suspension was seeded into a 96-well plate with 100 μL per well, and cultured overnight in a 37°C, 5% $CO_2$ incubator. After the cells fully adhered to the wall, 100 μL of the HMMW micropeptide and the four truncated polypeptides HMMW5-51, HMMW10-51, HMMW15-51, and HMMW20-51 having a concentration of 80 μM were added separately, and a culture solution without any drugs was used as a negative control group. After incubation was continued for 48 h, 100 μL of CCK8 was added per well in a 96-well plate for dissolution. An absorbance OD was measured at a detection wavelength of 450 nm using a microplate reader, and a proliferation inhibition (PI) was calculated using a formula: PI(%)=1-Treatment group/Negative control group. The experiment was independently repeated 3 times, and the results were expressed as Mean±SD. A statistical T-test was performed, and the results are shown in Table 2 and FIG. 2.

Table 2 Effects of different truncated fragments of micropeptide HMMW on proliferation of human tongue squamous cell carcinoma CAL27 cells

| Group | 48 h | |
|---|---|---|
| | OD | Proliferation inhibition (%) |
| Negative control group | 1.832±0.158 | - |
| HMMW | 0.351±0.078 | 80.841±4.258 |
| HMMW5-51 | 0.384±0.085 | 79.000±3.891 |
| HMMW10-51 | 0.426±0.093 | 76.747±5.076 |
| HMMW15-51 | 0.521±0.087 | 71.561±4.749 |

(continued)

| Group | 48 h | |
|---|---|---|
| | OD | Proliferation inhibition (%) |
| HMMW20-51 | 0.798±0.076 | 56.441±4.148** |
| Note: Compared with the HMMW treatment group, * represents *P*<0.05, and ** represents *P*<0.01. | | |

According to Table 2 and FIG. 2, compared with the HMMW treatment group, at the same dose, the three truncated polypeptides HMMW5-51, HMMW10-51, and HMMW15-51 have comparable inhibitory effects to that of the HMMW on proliferation of the human tongue squamous cell carcinoma CAL27 cells; and the truncated peptide HMMW20-51 has a significantly decreased inhibitory effect on proliferation of the human tongue squamous cell carcinoma CAL27 cells, but still has activity. Based on the results of Example 1, it is predicted that HMMW15-51 is the smallest functional fragment of the HMMW.

**Example 3**

This example provides alanine scan mutations of single amino acids in the micropeptide HMMW and affinity analysis.

Molecular docking simulation was performed on the micropeptide HMMW and a binding protein AQP2 thereof using MOE software, to identify predicted potential interaction sites. The docking simulation was divided into the following two groups: (1) random docking of an AQP2 homotetramer with the HMMW, without limiting a binding region; and (2) random docking of an AQP2 monomer with the HMMW, without limiting a binding region. Interaction sites obtained from the above two docking methods were integrated and analyzed, and all possible binding amino acids were selected as potential key sites for alanine (Ala) mutations. Due to alanine being the smallest chiral amino acid, the impact of a specific amino acid on an overall protein biological activity can be distinguished by systematically and sequentially substituting each amino acid in a sequence with Ala. The predicted polypeptides were obtained by solid-phase synthesis, and the affinity of different mutated polypeptides to the AQP2 protein was detected by microscale thermophoresis (MST) assay. The sequence information of the alanine scan mutations in the micropeptide HMMW is shown in FIG. 3, and the specific predicted key sites and the binding condition to the AQP2 are shown in Table 3.

Table 3 Prediction of key binding sites between micropeptide HMMW and AQP2 protein

| Polypeptide name | SEQ ID NO | Affinity (μM) |
|---|---|---|
| HMMW | SEQ ID NO.1 | 3.07 |
| HMMW-2A | SEQ ID NO.26 | 2.5 |
| HMMW-3A | SEQ ID NO.27 | 2.8 |
| HMMW-8A | SEQ ID NO.28 | 14.76 |
| HMMW-9A | SEQ ID NO.29 | 3.95 |
| HMMW-10A | SEQ ID NO.30 | 4.03 |
| HMMW-11A | SEQ ID NO.31 | 2.33 |
| HMMW-12A | SEQ ID NO.32 | 1.12 |
| HMMW-13A | SEQ ID NO.33 | 6.61 |
| HMMW-17A | SEQ ID NO.34 | 3.93 |
| HMMW-19A | SEQ ID NO.35 | 5.11 |
| HMMW-20A | SEQ ID NO.36 | 2.59 |
| HMMW-21A | SEQ ID NO.37 | 7.64 |
| HMMW-22A | SEQ ID NO.38 | 3.21 |
| HMMW-25A | SEQ ID NO.39 | 1.97 |
| HMMW-27A | SEQ ID NO.40 | 2.39 |
| HMMW-28A | SEQ ID NO.41 | 2.27 |

(continued)

| Polypeptide name | SEQ ID NO | Affinity (μM) |
|---|---|---|
| HMMW-30A | SEQ ID NO.42 | 2.23 |
| HMMW-31A | SEQ ID NO.43 | 3.06 |
| HMMW-32A | SEQ ID NO.44 | 2.01 |
| HMMW-40A | SEQ ID NO.45 | 1.19 |
| HMMW-41A | SEQ ID NO.46 | 4.32 |
| HMMW-42A | SEQ ID NO.47 | 0.25 |
| HMMW-43A | SEQ ID NO.48 | 0.36 |
| HMMW-44A | SEQ ID NO.49 | 5.23 |
| HMMW-45A | SEQ ID NO.50 | 3.24 |
| HMMW-48A | SEQ ID NO.51 | 11.64 |
| HMMW-49A | SEQ ID NO.52 | 20.76 |
| HMMW-50A | SEQ ID NO.53 | 0.55 |

According to Table 3, compared with the HMMW, the affinity of the polypeptide to the protein AQP2 significantly decreased following mutations of the amino acids at the positions 8, 48, and 49, preliminarily indicating that the key sites of the HMMW are the amino acids at the positions 8, 48, and 49.

**Example 4**

This example provides the effects of single amino acid mutation sequences of the micropeptide HMMW on proliferation of tongue squamous cell carcinoma CAL27 cells.

When human tongue squamous cell carcinoma CAL27 cells were cultured to a density of 90% in a 37°C, 5% $CO_2$ incubator, the cells were digested with trypsin and collected, resuspended in a culture solution, and counted under a microscope. The cell concentration was adjusted to approximately $5\times10^4$ cells/mL, and a cell suspension was seeded into a 96-well plate with 100 μL per well, and cultured overnight in a 37°C, 5% $CO_2$ incubator. After the cells fully adhered to the wall, 100 μL of the HMMW micropeptide and the single amino acid mutated polypeptide synthesized in Example 3 having a concentration of 80 μM were added separately, and a culture solution without any drugs was used as a negative control group. After incubation was continued for 48 h, 100 μL of CCK8 was added per well in a 96-well plate for dissolution. An absorbance OD was measured at a detection wavelength of 450 nm using a microplate reader, and a proliferation inhibition (PI) was calculated using a formula: PI(%)=1-Treatment group/Negative control group. The experiment was independently repeated 3 times, and the results were expressed as Mean±SD. A statistical T-test was performed, and the results are shown in FIG. 4, where * represents $P<0.05$, ** represents $P<0.01$, and *** represents $P<0.001$. At a concentration of 80 μM, except the two peptides HMMW-3A and HMMW-11A which have poor solubility and cannot conduct activity comparison, compared with the HMMW treatment group, the inhibitory effects of the polypeptides with the amino acids at the positions 43, 48, and 49 mutated on proliferation of the human tongue squamous cell carcinoma CAL27 cells were significantly decreased. The polypeptide with the amino acid at the position 8 mutated shows no difference in anti-tumor activity compared to the HMMW. Therefore, it is determined that the amino acids at the positions 43, 48, and 49 are the key active sites of the micropeptide HMMW.

**Example 5**

This example provides detection of inhibitory effects of the novel polypeptide HMMW15-51 (SEQ ID NO.21) and the micropeptide HMMW on proliferation of different tumor cells.

When human neuroblastoma cells SH-SY5Y, brain astrocytoma cells U87MG, oral epidermoid carcinoma cells KB, laryngeal epidermoid carcinoma cells HEp2, tongue squamous cell carcinoma cells CAL27, nasopharyngeal carcinoma cells CNE-2Z, esophageal cancer cells Eca-109 and TE-13, papillary thyroid carcinoma cells NPPA87-1, giant cell lung cancer cells 95-D, lung squamous cell carcinoma cells SK-MES-1, lung adenocarcinoma cells SPC-A1, non-small cell lung cancer cells A549, liver cancer cells HCCLM3 and HepG2, kidney cancer cells Ketr-3 and 786-O, breast cancer cells SK-BR-3 and MDA-MB-231, cervical cancer cells CaSki and Hela, uterine cancer cells RL-952 and MS751, ovarian cancer cells ES-2 and SKOV3, colon cancer cells HT29 and SW480, small intestine cancer cells HIC,

ileocecal cancer cells HCT-8, gastric cancer cells MGC-803 and BGC-823, bladder cancer cells T24 and EJ, pancreatic cancer cells ASPC-1 and PANC-1, prostate cancer cells 22RV1 and PC-3, cholangiocarcinoma cells QBC939, malignant melanoma cells SK-mel-2 and A375, osteosarcoma cells MG63, fibrosarcoma cells HT-1080, multiple myeloma cells LP-1, lymphoma cells Raji, peripheral blood B lymphocytes RPMI 8226 in multiple myeloma, histiocytic lymphoma cells U937, diffuse large B-cell lymphoma (DLBCL) cells WSU-DLCL2, acute lymphoblastic leukemia cells CEM/C1, acute promyelocytic leukemia cells NB4, acute myeloid leukemia cells KG-1, chronic myeloid leukemia cells K562, promyelocytic leukemia cells HL60, and T lymphoblastic leukemia cells (Jurkat, Clone E6-1) were cultured to a density of 90% in a 37°C, 5% $CO_2$ incubator, the cells were digested with trypsin and collected, resuspended in a culture solution, and counted under a microscope. The cell concentration was adjusted to approximately $1.0\times10^5$ cells/mL, and a cell suspension was seeded into a 96-well plate with 100 μL per well, and cultured overnight in a 37°C, 5% $CO_2$ incubator. After the cells fully adhered to the wall, 100 μL of the HMMW micropeptide having a concentration of 50 μM and the novel polypeptide HMMW15-51 having concentrations of 50 μM/75 μM/100 μM were added separately as a treatment group; and a culture solution without any drugs was used as a negative control group. After incubation was continued for 48 h, 10 μL of CCK8 was added per well in a 96-well plate for dissolution. Incubation was continued for 4 h in a cell incubator, an absorbance was measured at 450 nm, and a proliferation inhibition was calculated. Data was presented as mean ± SD, and GraphPad Prism 5.0 statistical software was used. A t-test was used for comparison between two groups, while One-way Anova (Dunnett) was used for comparison among a plurality of groups. The results are shown in Tables 4-56, where * represents $P<0.05$, ** represents $P<0.01$, and *** represents $P<0.001$.

Table 4. Inhibitory effects of novel polypeptide HMMW15-51 and micropeptide HMMW on proliferation of human brain astrocytoma cells U87MG

| Group | | 48 h | |
|---|---|---|---|
| | | OD | Inhibition (%) |
| Negative control group | | 0.965±0.026 | - |
| Novel polypeptide HMMW15-51 | 100 μM | 0.112±0.024** | 88.359±2.440** |
| | 75 μM | 0.169±0.023** | 82.487±2.383** |
| | 50 μM | 0.197±0.042** | 79.585±4.324** |
| HMMW | 50 μM | 0.365±0.019** | 62.176±2.001** |

Table 5. Inhibitory effects of novel polypeptide HMMW15-51 and micropeptide HMMW on proliferation of human neuroblastoma cells SH-SY5Y

| Group | | 48 h | |
|---|---|---|---|
| | | OD | Inhibition (%) |
| Negative control group | | 0.983±0.015 | - |
| Novel polypeptide HMMW15-51 | 100 μM | 0.162±0.019 | 83.525±1.948** |
| | 75 μM | 0.191±0.037 | 80.576±3.787** |
| | 50 μM | 0.263±0.034 | 73.220±3.443** |
| HMMW | 50 μM | 0.486±0.049 | 50.542±4.982** |

Table 6. Inhibitory effects of novel polypeptide HMMW15-51 and micropeptide HMMW on proliferation of human oral epidermoid carcinoma cells KB

| Group | | 48 h | |
|---|---|---|---|
| | | OD | Inhibition (%) |
| Negative control group | | 1.010±0.021 | - |
| Novel polypeptide HMMW15-51 | 100 μM | 0.122±0.026** | 87.888±2.575** |
| | 75 μM | 0.167±0.005** | 83.432±0.508** |
| | 50 μM | 0.218±0.012** | 78.383±1.148** |

(continued)

| Group | | 48 h | |
|---|---|---|---|
| | | OD | Inhibition (%) |
| HMMW | 50 μM | 0.471±0.040** | 53.399±3.971** |

Table 7. Inhibitory effects of novel polypeptide HMMW15-51 and micropeptide HMMW on proliferation of human laryngeal epidermoid carcinoma cells HEp2

| Group | | 48 h | |
|---|---|---|---|
| | | OD | Inhibition (%) |
| Negative control group | | 0.875±0.011 | - |
| Novel polypeptide HMMW15-51 | 100 μM | 0.127±0.007** | 85.518±0.744** |
| | 75 μM | 0.169+0.027** | 80.716±3.030** |
| | 50 μM | 0.210±0.024** | 76.029±2.728** |
| HMMW | 50 μM | 0.504±0.020** | 42.416±2.236** |

Table 8. Inhibitory effects of novel polypeptide HMMW15-51 and micropeptide HMMW on proliferation of human tongue squamous cell carcinoma cells CAL27

| Group | | 48 h | |
|---|---|---|---|
| | | OD | Inhibition (%) |
| Negative control group | | 1.165±0.012 | - |
| Novel polypeptide HMMW15-51 | 100 μM | 0.151±0.010** | 87.035±0.828** |
| | 75 μM | 0.211±0.039** | 81.912±3.381** |
| | 50 μM | 0.261±0.021** | 77.619±1.804** |
| HMMW | 50 μM | 0.383±0.064** | 67.115±5.457** |

Table 9. Inhibitory effects of novel polypeptide HMMW15-51 and micropeptide HMMW on proliferation of human nasopharyngeal carcinoma cells CNE-2Z

| Group | | 48 h | |
|---|---|---|---|
| | | OD | Inhibition (%) |
| Negative control group | | 0.963±0.023 | - |
| Novel polypeptide HMMW15-51 | 100 μM | 0.112±0.012** | 88.404±1.252** |
| | 75 μM | 0.154±0.017** | 84.043±1.806** |
| | 50 μM | 0.244±0.044** | 74.697±4.553** |
| HMMW | 50 μM | 0.472±0.042** | 50.952±4.312** |

Table 10. Inhibitory effects of novel polypeptide HMMW15-51 and micropeptide HMMW on proliferation of human esophageal cancer cells Eca-109

| Group | | 48 h | |
|---|---|---|---|
| | | OD | Inhibition (%) |
| Negative control group | | 0.949±0.041 | - |
| Novel polypeptide HMMW15-51 | 100 μM | 0.076±0.017** | 92.024±1.793** |
| | 75 μM | 0.109±0.025** | 88.510±2.588** |
| | 50 μM | 0.168±0.034** | 82.256±3.531** |

(continued)

| Group | | 48 h | |
|---|---|---|---|
| | | OD | Inhibition (%) |
| HMMW | 50 μM | 0.350±0.028** | 63.071±2.982** |

Table 11. Inhibitory effects of novel polypeptide HMMW15-51 and micropeptide HMMW on proliferation of human esophageal cancer cells TE-13

| Group | | 48 h | |
|---|---|---|---|
| | | OD | Inhibition (%) |
| Negative control group | | 0.828±0.017 | - |
| Novel polypeptide HMMW15-51 | 100 μM | 0.132±0.012** | 84.018±1.451** |
| | 75 μM | 0.205±0.013** | 75.242±1.579** |
| | 50 μM | 0.302±0.029** | 63.486±3.547** |
| HMMW | 50 μM | 0.389±0.016** | 53.060±1.911** |

Table 12. Inhibitory effects of novel polypeptide HMMW15-51 and micropeptide HMMW on proliferation of human papillary thyroid carcinoma cells NPPA87-1

| Group | | 48 h | |
|---|---|---|---|
| | | OD | Inhibition (%) |
| Negative control group | | 0.922±0.032 | - |
| Novel polypeptide HMMW15-51 | 100 μM | 0.221±0.019** | 76.030±2.061** |
| | 75 μM | 0.271±0.044** | 70.607±4.793** |
| | 50 μM | 0.381±0.026** | 58.677±2.767** |
| HMMW | 50 μM | 0.478±0.028** | 48.192±2.999** |

Table 13. Inhibitory effects of novel polypeptide HMMW15-51 and micropeptide HMMW on proliferation of human giant cell lung cancer cells 95-D

| Group | | 48 h | |
|---|---|---|---|
| | | OD | Inhibition (%) |
| Negative control group | | 1.098±0.012 | - |
| Novel polypeptide HMMW15-51 | 100 μM | 0.150±0.018** | 86.343±1.600** |
| | 75 μM | 0.211±0.020** | 80.789±1.791** |
| | 50 μM | 0.259±0.032** | 76.449±2.914** |
| HMMW | 50 μM | 0.442±0.059** | 59.757±5.365** |

Table 14. Inhibitory effects of novel polypeptide HMMW15-51 and micropeptide HMMW on proliferation of human lung squamous cell carcinoma cells SK-MES-1

| Group | | 48 h | |
|---|---|---|---|
| | | OD | Inhibition (%) |
| Negative control group | | 0.964±0.024 | - |
| Novel polypeptide HMMW15-51 | 100 μM | 0.119±0.024** | 87.621±2.501** |
| | 75 μM | 0.203±0.030** | 78.942±3.061** |
| | 50 μM | 0.279±0.018** | 71.058±1.844** |

(continued)

| Group | | 48 h | |
|---|---|---|---|
| | | OD | Inhibition (%) |
| HMMW | 50 μM | 0.435±0.043** | 54.910±4.426** |

Table 15. Inhibitory effects of novel polypeptide HMMW15-51 and micropeptide HMMW on proliferation of human lung adenocarcinoma cells SPC-A1

| Group | | 48 h | |
|---|---|---|---|
| | | OD | Inhibition (%) |
| Negative control group | | 1.032±0.038 | - |
| Novel polypeptide HMMW15-51 | 100 μM | 0.151±0.028** | 85.368±2.729** |
| | 75 μM | 0.259±0.018** | 74.935±1.734** |
| | 50 μM | 0.358±0.044** | 65.278±4.300** |
| HMMW | 50 μM | 0.525±0.032** | 49.128±3.053** |

Table 16. Inhibitory effects of novel polypeptide HMMW15-51 and micropeptide HMMW on proliferation of human non-small cell lung cancer cells A549

| Group | | 48 h | |
|---|---|---|---|
| | | OD | Inhibition (%) |
| Negative control group | | 1.079±0.022 | - |
| Novel polypeptide HMMW15-51 | 100 μM | 0.229±0.041** | 78.808±3.770** |
| | 75 μM | 0.354±0.041** | 67.161±3.844** |
| | 50 μM | 0.469±0.003** | 56.534±0.278** |
| HMMW | 50 μM | 0.508±0.022** | 52.950±2.023** |

Table 17. Inhibitory effects of novel polypeptide HMMW15-51 and micropeptide HMMW on proliferation of human liver cancer cells HCCLM3

| Group | | 48 h | |
|---|---|---|---|
| | | OD | Inhibition (%) |
| Negative control group | | 0.998±0.032 | - |
| Novel polypeptide HMMW15-51 | 100 μM | 0.117±0.025** | 88.239±2.474** |
| | 75 μM | 0.201±0.037** | 79.820±3.700** |
| | 50 μM | 0.301±0.039** | 69.863±3.920** |
| HMMW | 50 μM | 0.460±0.076** | 53.926±7.634** |

Table 18. Inhibitory effects of novel polypeptide HMMW15-51 and micropeptide HMMW on proliferation of human liver cancer cells HepG2

| Group | | 48 h | |
|---|---|---|---|
| | | OD | Inhibition (%) |
| Negative control group | | 1.073±0.036 | - |
| Novel polypeptide HMMW15-51 | 100 μM | 0.154±0.022** | 85.674±2.069** |
| | 75 μM | 0.241±0.019** | 77.564±1.730** |
| | 50 μM | 0.311±0.052** | 70.976±4.848** |

(continued)

| Group | | 48 h | |
|---|---|---|---|
| | | OD | Inhibition (%) |
| HMMW | 50 μM | 0.509±0.027** | 52.579±2.504** |

Table 19. Inhibitory effects of novel polypeptide HMMW15-51 and micropeptide HMMW on proliferation of human kidney cancer cells Ketr-3

| Group | | 48 h | |
|---|---|---|---|
| | | OD | Inhibition (%) |
| Negative control group | | 0.929±0.049 | - |
| Novel polypeptide HMMW15-51 | 100 μM | 0.178±0.016** | 80.797±1.717** |
| | 75 μM | 0.248±0.026** | 73.295±2.760** |
| | 50 μM | 0.351±0.020** | 62.240±2.197** |
| HMMW | 50 μM | 0.476±0.045** | 48.708±4.852** |

Table 20. Inhibitory effects of novel polypeptide HMMW15-51 and micropeptide HMMW on proliferation of human kidney clear cell adenocarcinoma cells 786-O

| Group | | 48 h | |
|---|---|---|---|
| | | OD | Inhibition (%) |
| Negative control group | | 1.105±0.019 | - |
| Novel polypeptide HMMW15-51 | 100 μM | 0.205±0.035** | 81.478±3.122** |
| | 75 μM | 0.312±0.029** | 71.765±2.634** |
| | 50 μM | 0.341±0.054** | 69.140±4.893** |
| HMMW | 50 μM | 0.413±0.078** | 62.624±7.014** |

Table 21. Inhibitory effects of novel polypeptide HMMW15-51 and micropeptide HMMW on proliferation of human breast cancer cells SK-BR-3

| Group | | 48 h | |
|---|---|---|---|
| | | OD | Inhibition (%) |
| Negative control group | | 1.096±0.039 | - |
| Novel polypeptide HMMW15-51 | 100 μM | 0.122±0.019** | 88.899±1.746** |
| | 75 μM | 0.224±0.049** | 79.592±4.474** |
| | 50 μM | 0.291±0.022** | 73.418±1.970** |
| HMMW | 50 μM | 0.507±0.054** | 53.710±4.902** |

Table 22. Inhibitory effects of novel polypeptide HMMW15-51 and micropeptide HMMW on proliferation of human breast cancer cells MDA-MB-231

| Group | | 48 h | |
|---|---|---|---|
| | | OD | Inhibition (%) |
| Negative control group | | 1.233±0.007 | - |
| Novel polypeptide HMMW15-51 | 100 μM | 0.211±0.027** | 82.865±2.194** |
| | 75 μM | 0.237±0.032** | 80.757±2.620** |
| | 50 μM | 0.341±0.042** | 72.378±3.406** |

(continued)

| Group | | 48 h | |
|---|---|---|---|
| | | OD | Inhibition (%) |
| HMMW | 50 μM | 0.429±0.056$^{**}$ | 65.216±4.512$^{**}$ |

Table 23. Inhibitory effects of novel polypeptide HMMW15-51 and micropeptide HMMW on proliferation of human cervical cancer epithelial cells CaSki

| Group | | 48 h | |
|---|---|---|---|
| | | OD | Inhibition (%) |
| Negative control group | | 0.981±0.035 | - |
| Novel polypeptide HMMW15-51 | 100 μM | 0.228±0.035$^{**}$ | 76.800±3.597$^{**}$ |
| | 75 μM | 0.302±0.030$^{**}$ | 69.226±3.007$^{**}$ |
| | 50 μM | 0.402±0.024$^{**}$ | 59.001±2.408$^{**}$ |
| HMMW | 50 μM | 0.458±0.038$^{**}$ | 53.329±3.884$^{**}$ |

Table 24. Inhibitory effects of novel polypeptide HMMW15-51 and micropeptide HMMW on proliferation of human cervical cancer cells Hela

| Group | | 48 h | |
|---|---|---|---|
| | | OD | Inhibition (%) |
| Negative control group | | 0.964±0.023 | - |
| Novel polypeptide HMMW15-51 | 100 μM | 0.198±0.026$^{**}$ | 83.661±1.557$^{**}$ |
| | 75 μM | 0.328±0.011$^{**}$ | 74.508±2.703$^{**}$ |
| | 50 μM | 0.377±0.044$^{**}$ | 66.508±2.412$^{**}$ |
| HMMW | 50 μM | 0.493±0.049$^{**}$ | 52.644±4.018$^{**}$ |

Table 25. Inhibitory effects of novel polypeptide HMMW15-51 and micropeptide HMMW on proliferation of human endometrial cancer cells RL-952

| Group | 48 h | | |
|---|---|---|---|
| | OD | Inhibition (%) | |
| Negative control group | 0.860±0.027 | - | |
| Novel polypeptide HMMW15-51 | 100 μM | 0.190±0.038$^{**}$ | 77.916±4.415$^{**}$ |
| | 75 μM | 0.324±0.022$^{**}$ | 62.379±2.505$^{**}$ |
| | 50 μM | 0.355±0.044$^{**}$ | 58.776±5.167$^{**}$ |
| HMMW | 50 μM | 0.422±0.034$^{**}$ | 50.988±3.897$^{**}$ |

Table 26. Inhibitory effects of novel polypeptide HMMW15-51 and micropeptide HMMW on proliferation of human uterine epidermal cancer cells MS751

| Group | | 48 h | |
|---|---|---|---|
| | | OD | Inhibition (%) |
| Negative control group | | 0.934±0.013 | - |
| Novel polypeptide HMMW15-51 | 100 μM | 0.259±0.026$^{**}$ | 72.296±2.763$^{**}$ |
| | 75 μM | 0.355±0.024$^{**}$ | 61.942±2.517$^{**}$ |
| | 50 μM | 0.430±0.031$^{**}$ | 53.945±3.268$^{**}$ |

(continued)

| Group | | 48 h | |
|---|---|---|---|
| | | OD | Inhibition (%) |
| HMMW | 50 μM | 0.474±0.024** | 49.268±2.531** |

Table 27. Inhibitory effects of novel polypeptide HMMW15-51 and micropeptide HMMW on proliferation of human ovarian clear cell carcinoma cells ES-2

| Group | | 48 h | |
|---|---|---|---|
| | | OD | Inhibition (%) |
| Negative control group | | 0.966±0.032 | - |
| Novel polypeptide HMMW15-51 | 100 μM | 0.183±0.013** | 81.049±1.298** |
| | 75 μM | 0.270±0.028** | 72.006±2.904** |
| | 50 μM | 0.410±0.042** | 57.508±4.384** |
| HMMW | 50 μM | 0.464±0.007** | 51.916±0.690** |

Table 28. Inhibitory effects of novel polypeptide HMMW15-51 and micropeptide HMMW on proliferation of human ovarian cancer cells SKOV3

| Group | | 48 h | |
|---|---|---|---|
| | | OD | Inhibition (%) |
| Negative control group | | 0.983±0.042 | - |
| Novel polypeptide HMMW15-51 | 100 μM | 0.161±0.015** | 83.661±1.557** |
| | 75 μM | 0.251±0.027** | 74.508±2.703** |
| | 50 μM | 0.329±0.024** | 66.508±2.412** |
| HMMW | 50 μM | 0.466±0.040** | 52.644±4.018** |

Table 29. Inhibitory effects of novel polypeptide HMMW15-51 and micropeptide HMMW on proliferation of human colon cancer cells HT29

| Group | | 48 h | |
|---|---|---|---|
| | | OD | Inhibition (%) |
| Negative control group | | 0.974±0.016 | - |
| Novel polypeptide HMMW15-51 | 100 μM | 0.103±0.019** | 89.456±1.928** |
| | 75 μM | 0.129±0.014** | 86.717±1.402** |
| | 50 μM | 0.192±0.006** | 80.246±0.584** |
| HMMW | 50 μM | 0.479±0.024** | 50.805±2.422** |

Table 30. Inhibitory effects of novel polypeptide HMMW15-51 and micropeptide HMMW on proliferation of human colon cancer cells SW480

| Group | | 48 h | |
|---|---|---|---|
| | | OD | Inhibition (%) |
| Negative control group | | 0.941±0.028 | - |
| Novel polypeptide HMMW15-51 | 100 μM | 0.160±0.035** | 82.991±3.678** |
| | 75 μM | 0.269±0.029** | 71.439±3.113** |
| | 50 μM | 0.358±0.016** | 61.906±1.695** |

(continued)

| Group | | 48 h | |
|---|---|---|---|
| | | OD | Inhibition (%) |
| HMMW | 50 μM | 0.479±0.025** | 49.114±2.626** |

Table 31. Inhibitory effects of novel polypeptide HMMW15-51 and micropeptide HMMW on proliferation of human small intestine cancer cells HIC

| Group | | 48 h | |
|---|---|---|---|
| | | OD | Inhibition (%) |
| Negative control group | | 1.018±0.018 | - |
| Novel polypeptide HMMW15-51 | 100 μM | 0.221±0.040** | 78.251±3.911** |
| | 75 μM | 0.293±0.022** | 71.209±2.133** |
| | 50 μM | 0.383±0.031** | 62.332±3.021** |
| HMMW | 50 μM | 0.507±0.022** | 50.213±2.165** |

Table 32. Inhibitory effects of novel polypeptide HMMW15-51 and micropeptide HMMW on proliferation of human ileocecal cancer cells HCT-8

| Group | 48 h | | |
|---|---|---|---|
| | | OD | Inhibition (%) |
| Negative control group | | 0.939±0.029 | - |
| Novel polypeptide HMMW15-51 | 100 μM | 0.205±0.047** | 78.211±4.966** |
| | 75 μM | 0.297±0.066** | 68.346±7.028** |
| | 50 μM | 0.445±0.064** | 52.590±6.777** |
| HMMW | 50 μM | 0.482±0.036** | 48.687±3.785** |

Table 33. Inhibitory effects of novel polypeptide HMMW15-51 and micropeptide HMMW on proliferation of human gastric cancer cells MGC-803

| Group | | 48 h | |
|---|---|---|---|
| | | OD | Inhibition (%) |
| Negative control group | | 1.145±0.029 | - |
| Novel polypeptide HMMW15-51 | 100 μM | 0.134±0.010** | 88.329±0.861** |
| | 75 μM | 0.190±0.020** | 83.382±1.747** |
| | 50 μM | 0.254±0.010** | 77.852±0.861** |
| HMMW | 50 μM | 0.675±0.035** | 41.094±3.077** |

Table 34. Inhibitory effects of novel polypeptide HMMW15-51 and micropeptide HMMW on proliferation of human gastric cancer cells BGC-823

| Group | | 48 h | |
|---|---|---|---|
| | | OD | Inhibition (%) |
| Negative control group | | 0.976±0.038 | - |
| Novel polypeptide HMMW15-51 | 100 μM | 0.169±0.021** | 82.690±2.158** |
| | 75 μM | 0.229±0.027** | 76.511±2.776** |
| | 50 μM | 0.299±0.037** | 69.409±3.800** |

(continued)

| Group | | 48 h | |
|---|---|---|---|
| | | OD | Inhibition (%) |
| HMMW | 50 μM | 0.510±0.046** | 47.730±4.739** |

Table 35. Inhibitory effects of novel polypeptide HMMW15-51 and micropeptide HMMW on proliferation of human bladder transitional cell carcinoma cells T24

| Group | | 48 h | |
|---|---|---|---|
| | | OD | Inhibition (%) |
| Negative control group | | 0.950±0.013 | - |
| Novel polypeptide HMMW15-51 | 100 μM | 0.090±0.013** | 90.523±1.319** |
| | 75 μM | 0.158±0.033** | 83.363±3.522** |
| | 50 μM | 0.205±0.011** | 78.378±1.184** |
| HMMW | 50 μM | 0.460±0.045** | 51.562±4.780** |

Table 36. Inhibitory effects of novel polypeptide HMMW15-51 and micropeptide HMMW on proliferation of human bladder cancer cells EJ

| Group | | 48 h | |
|---|---|---|---|
| | | OD | Inhibition (%) |
| Negative control group | | 1.223±0.018 | - |
| Novel polypeptide HMMW15-51 | 100 μM | 0.169±0.052** | 86.185±4.258** |
| | 75 μM | 0.345±0.017** | 71.798±1.375** |
| | 50 μM | 0.439±0.016** | 64.142±1.270** |
| HMMW | 50 μM | 0.638±0.018** | 47.820±1.486** |

Table 37. Inhibitory effects of novel polypeptide HMMW15-51 and micropeptide HMMW on proliferation of human pancreatic cancer cells ASPC-1

| Group | | 48 h | |
|---|---|---|---|
| | | OD | Inhibition (%) |
| Negative control group | | 1.024±0.020** | - |
| Novel polypeptide HMMW15-51 | 100 μM | 0.131±0.027** | 87.179±2.645** |
| | 75 μM | 0.234±0.035** | 77.188±3.413** |
| | 50 μM | 0.363±0.063** | 64.562±6.146** |
| HMMW | 50 μM | 0.497±0.045** | 51.448±4.377** |

Table 38. Inhibitory effects of novel polypeptide HMMW15-51 and micropeptide HMMW on proliferation of human pancreatic cancer cells PANC-1

| Group | | 48 h | |
|---|---|---|---|
| | | OD | Inhibition (%) |
| Negative control group | | 0.853±0.034 | - |
| Novel polypeptide HMMW15-51 | 100 μM | 0.095±0.013** | 88.906±1.493** |
| | 75 μM | 0.184±0.023** | 78.398±2.704** |
| | 50 μM | 0.310±0.047** | 63.672±5.525** |

(continued)

| Group | | 48 h | |
|---|---|---|---|
| | | OD | Inhibition (%) |
| HMMW | 50 μM | 0.424±0.052** | 50.273±6.135** |

Table 39. Inhibitory effects of novel polypeptide HMMW15-51 and micropeptide HMMW on proliferation of human prostate cancer cells 22RV1

| Group | | 48 h | |
|---|---|---|---|
| | | OD | Inhibition (%) |
| Negative control group | | 1.115±0.030 | - |
| Novel polypeptide HMMW15-51 | 100 μM | 0.193±0.042** | 82.720±3.730** |
| | 75 μM | 0.305±0.044** | 72.646±3.906** |
| | 50 μM | 0.426±0.036** | 61.794±3.210** |
| HMMW | 50 μM | 0.552±0.019** | 50.463±1.693** |

Table 40. Inhibitory effects of novel polypeptide HMMW15-51 and micropeptide HMMW on proliferation of human prostate cancer cells PC-3

| Group | | 48 h | |
|---|---|---|---|
| | | OD | Inhibition (%) |
| Negative control group | | 1.025±0.014 | - |
| Novel polypeptide HMMW15-51 | 100 μM | 0.193±0.049** | 81.203±4.742** |
| | 75 μM | 0.244±0.017** | 76.163±1.651** |
| | 50 μM | 0.291±0.038** | 71.642±3.715** |
| HMMW | 50 μM | 0.509±0.040** | 50.341±3.864** |

Table 41. Inhibitory effects of novel polypeptide HMMW15-51 and micropeptide HMMW on proliferation of human cholangiocarcinoma cells QBC939

| Group | | 48 h | |
|---|---|---|---|
| | | OD | Inhibition (%) |
| Negative control group | | 0.850±0.032 | - |
| Novel polypeptide HMMW15-51 | 100 μM | 0.156±0.037** | 81.608±4.366** |
| | 75 μM | 0.253±0.041** | 70.196±4.847** |
| | 50 μM | 0.344±0.037** | 59.529±4.305** |
| HMMW | 50 μM | 0.429±0.018** | 49.490±2.138** |

Table 42. Inhibitory effects of novel polypeptide HMMW15-51 and micropeptide HMMW on proliferation of human malignant melanoma cells SK-mel-2

| Group | | 48 h | |
|---|---|---|---|
| | | OD | Inhibition (%) |
| Negative control group | | 0.867±0.029 | - |
| Novel polypeptide HMMW15-51 | 100 μM | 0.133±0.025** | 84.704±2.846** |
| | 75 μM | 0.174±0.019** | 79.977±2.225** |
| | 50 μM | 0.233±0.014** | 73.136±1.614** |

(continued)

| Group | | 48 h | |
|---|---|---|---|
| | | OD | Inhibition (%) |
| HMMW | 50 μM | 0.466±0.042** | 46.311±4.884** |

Table 43. Inhibitory effects of novel polypeptide HMMW15-51 and micropeptide HMMW on proliferation of human malignant melanoma cells A375

| Group | | 48 h | |
|---|---|---|---|
| | | OD | Inhibition (%) |
| Negative control group | | 0.974±0.035 | - |
| Novel polypeptide HMMW15-51 | 100 μM | 0.150±0.045** | 84.571±4.579** |
| | 75 μM | 0.269±0.058** | 72.357±5.914** |
| | 50 μM | 0.360±0.015** | 63.052±1.491** |
| HMMW | 50 μM | 0.491±0.040** | 49.572±4.128** |

Table 44. Inhibitory effects of novel polypeptide HMMW15-51 and micropeptide HMMW on proliferation of human osteosarcoma cells MG63

| Group | | 48 h | |
|---|---|---|---|
| | | OD | Inhibition (%) |
| Negative control group | | 0.917±0.032 | - |
| Novel polypeptide HMMW15-51 | 100 μM | 0.126±0.025** | 86.228±2.728** |
| | 75 μM | 0.200±0.023** | 78.161±2.520** |
| | 50 μM | 0.270±0.026** | 70.603±2.845** |
| HMMW | 50 μM | 0.435±0.024** | 52.544±2.562** |

Table 45. Inhibitory effects of novel polypeptide HMMW15-51 and micropeptide HMMW on proliferation of human fibrosarcoma cells HT-1080

| Group | | 48 h | |
|---|---|---|---|
| | | OD | Inhibition (%) |
| Negative control group | | 0.922±0.030 | - |
| Novel polypeptide HMMW15-51 | 100 μM | 0.166±0.022** | 81.953±2.407** |
| | 75 μM | 0.256±0.036** | 72.260±3.852** |
| | 50 μM | 0.365±0.032** | 60.434±3.432** |
| HMMW | 50 μM | 0.432±0.038** | 53.092±4.138** |

Table 46. Inhibitory effects of novel polypeptide HMMW15-51 and micropeptide HMMW on proliferation of human multiple myeloma cells LP-1

| Group | | 48 h | |
|---|---|---|---|
| | | OD | Inhibition (%) |
| Negative control group | | 0.882±0.037 | - |
| Novel polypeptide HMMW15-51 | 100 μM | 0.146±0.027** | 83.440±3.052** |
| | 75 μM | 0.200±0.009** | 77.278±1.054** |
| | 50 μM | 0.299±0.023** | 66.125±2.629** |

(continued)

| Group | | 48 h | |
|---|---|---|---|
| | | OD | Inhibition (%) |
| HMMW | 50 μM | 0.462±0.038** | 47.561±4.259** |

Table 47. Inhibitory effects of novel polypeptide HMMW15-51 and micropeptide HMMW on proliferation of human lymphoma cells Raji

| Group | | 48 h | |
|---|---|---|---|
| | | OD | Inhibition (%) |
| Negative control group | | 1.268±0.042 | - |
| Novel polypeptide HMMW15-51 | 100 μM | 0.189±0.017** | 85.099±1.317** |
| | 75 μM | 0.237±0.020** | 81.340±1.546** |
| | 50 μM | 0.380±0.036** | 70.013±2.862** |
| HMMW | 50 μM | 0.576±0.047** | 54.560±3.675** |

Table 48. Inhibitory effects of novel polypeptide HMMW15-51 and micropeptide HMMW on proliferation of human peripheral blood B lymphocytes RPMI 8226 in multiple myeloma

| Group | | 48 h | |
|---|---|---|---|
| | | OD | Inhibition (%) |
| Negative control group | | 1.229±0.022 | - |
| Novel polypeptide HMMW15-51 | 100 μM | 0.148±0.025** | 87.954±2.000** |
| | 75 μM | 0.235±0.011** | 80.846±0.908** |
| | 50 μM | 0.360±0.034** | 70.700±2.799** |
| HMMW | 50 μM | 0.578±0.056** | 52.930±4.562** |

Table 49. Inhibitory effects of novel polypeptide HMMW15-51 and micropeptide HMMW on proliferation of human histiocytic lymphoma cells U937

| Group | | 48 h | |
|---|---|---|---|
| | | OD | Inhibition (%) |
| Negative control group | | 1.337±0.034 | - |
| Novel polypeptide HMMW15-51 | 100 μM | 0.331±0.031** | 75.274±2.328** |
| | 75 μM | 0.498±0.050** | 62.787±3.714** |
| | 50 μM | 0.599±0.044** | 55.234±3.264** |
| HMMW | 50 μM | 0.855±0.029** | 36.042±2.134** |

Table 50. Inhibitory effects of novel polypeptide HMMW15-51 and micropeptide HMMW on proliferation of human diffuse large B-cell lymphoma (DLBCL) cells WSU-DLCL2

| Group | | 48 h | |
|---|---|---|---|
| | | OD | Inhibition (%) |
| Negative control group | | 1.313±0.040 | - |
| Novel polypeptide HMMW15-51 | 100 μM | 0.277±0.028** | 78.934±2.143** |
| | 75 μM | 0.478±0.032** | 63.629±2.463** |
| | 50 μM | 0.626±0.048** | 52.360±3.672** |

(continued)

| Group | | 48 h | |
|---|---|---|---|
| | | OD | Inhibition (%) |
| HMMW | 50 μM | 0.731±0.032** | 44.365±2.462** |

Table 51. Inhibitory effects of novel polypeptide HMMW15-51 and micropeptide HMMW on proliferation of human acute lymphoblastic leukemia cells CEM/C1

| Group | | 48 h | |
|---|---|---|---|
| | | OD | Inhibition (%) |
| Negative control group | | 1.177+0.035 | - |
| Novel polypeptide HMMW15-51 | 100 μM | 0.251±0.043** | 78.709±3.692** |
| | 75 μM | 0.443±0.050** | 62.344±4.216** |
| | 50 μM | 0.579±0.059** | 50.849±5.040** |
| HMMW | 50 μM | 0.630±0.017** | 46.518±1.484** |

Table 52. Inhibitory effects of novel polypeptide HMMW15-51 and micropeptide HMMW on proliferation of human acute promyelocytic leukemia cells NB4

| Group | | 48 h | |
|---|---|---|---|
| | | OD | Inhibition (%) |
| Negative control group | | 1.415±0.037 | - |
| Novel polypeptide HMMW15-51 | 100 μM | 0.354±0.047** | 74.988±3.332** |
| | 75 μM | 0.504±0.040** | 64.390±2.797** |
| | 50 μM | 0.685±0.019** | 51.578±1.363** |
| HMMW | 50 μM | 0.740±0.070** | 47.692±4.919** |

Table 53. Inhibitory effects of novel polypeptide HMMW15-51 and micropeptide HMMW on proliferation of human acute myeloid leukemia cells KG-1

| Group | | 48 h | |
|---|---|---|---|
| | | OD | Inhibition (%) |
| Negative control group | | 1.161±0.046 | - |
| Novel polypeptide HMMW15-51 | 100 μM | 0.319±0.033** | 72.532±2.815** |
| | 75 μM | 0.420±0.016** | 63.863±1.416** |
| | 50 μM | 0.561±0.017** | 51.665±1.467** |
| HMMW | 50 μM | 0.725±0.070** | 37.600±5.989** |

Table 54. Inhibitory effects of novel polypeptide HMMW15-51 and micropeptide HMMW on proliferation of human chronic myeloid leukemia cells K562

| Group | | 48 h | |
|---|---|---|---|
| | | OD | Inhibition (%) |
| Negative control group | | 1.285±0.026 | - |
| Novel polypeptide HMMW15-51 | 100 μM | 0.409±0.052** | 68.197±4.053** |
| | 75 μM | 0.524±0.038** | 59.222±2.991** |
| | 50 μM | 0.597±0.046** | 53.567±3.588** |

(continued)

| Group | | 48 h | |
|---|---|---|---|
| | | OD | Inhibition (%) |
| HMMW | 50 μM | 0.618±0.036** | 5l.933±2.838** |

Table 55. Inhibitory effects of novel polypeptide HMMW15-51 and micropeptide HMMW on proliferation of human promyelocytic leukemia cells HL60

| Group | | 48 h | |
|---|---|---|---|
| | | OD | Inhibition (%) |
| Negative control group | | 1.097±0.035 | - |
| Novel polypeptide HMMW15-51 | 100 μM | 0.310±0.041** | 71.733±3.732** |
| | 75 μM | 0.427±0.025** | 61.064±2.269** |
| | 50 μM | 0.501±0.008** | 54.286±0.696** |
| HMMW | 50 μM | 0.578±0.062** | 47.264±5.692** |

Table 56. Inhibitory effects of novel polypeptide HMMW15-51 and micropeptide HMMW on proliferation of human T lymphoblastic leukemia cells Jurkat, Clone E6-1

| Group | | 48 h | |
|---|---|---|---|
| | | OD | Inhibition (%) |
| Negative control group | | 1.080±0.029 | - |
| Novel polypeptide HMMW15-51 | 100 μM | 0.275±0.053** | 74.545±4.881** |
| | 75 μM | 0.406±0.051** | 62.388±4.707** |
| | 50 μM | 0.486±0.059** | 55.014±5.442** |
| HMMW | 50 μM | 0.602±0.037** | 44.307±3.404** |

According to Tables 4-56 that at the same dose, the novel polypeptide HMMW15-51 has significantly better inhibitory effects on proliferation of neuroblastoma, human glioma, head and neck cancer, esophageal cancer, thyroid cancer, lung cancer, liver cancer, kidney cancer, breast cancer, cervical cancer, uterine cancer, ovarian cancer, intestinal cancer, gastric cancer, bladder cancer, pancreatic cancer, prostate cancer, cholangiocarcinoma, melanoma, osteosarcoma, fibrosarcoma, myeloma, lymphoma, and hematoma cells than the micropeptide HMMW. Moreover, the inhibitory effects of the novel polypeptide HMMW15-51 on proliferation of the tumor cells showed a dose-dependent relationship, indicating that the novel polypeptide HMMW15-51 may be developed into anti-tumor drugs by inhibiting proliferation of the malignant tumor cells.

**Example 6**

This example provides inhibitory effects of the novel polypeptide HMMW15-51 (SEQ ID NO.21) and the micropeptide HMMW on metastasis of different human tumor cells.

Melanoma, cervical cancer, ovarian cancer, liver cancer, and lung cancer cells were seeded into transwell chambers, with 100 μL per well. Different doses of the HMMW micropeptide and the novel polypeptide HMMW15-51 were added to each chamber as a treatment group, while no drug was added to a blank group. Then, 0.6 mL of a complete culture medium containing 10% FBS was added to the lower chambers of the transwell to stimulate cell metastasis, and the cells were cultured at 5% $CO_2$ and 37°C for 48 h. The culture solution was discarded from the wells. The cells were fixed with methanol at room temperature for 30 min, stained with 0.1% crystal violet at room temperature for 10 min, and rinsed with clean water. The upper layer nonmetastatic cells were wiped off with a cotton swab. The cells were observed under a microscope, and four fields of view were selected to take photos and count the cells. A metastasis inhibition (MI) was calculated according to a formula:

$$MI(\%) = \left(1 - \frac{N_{test}}{N_{control}}\right) \times 100\%,$$

where $N_{test}$ represents the number of cell metastases in the treatment group, and $N_{control}$ represents the number of cell metastases in the blank control group.

Data was presented as mean ± SD, and GraphPad Prism 5.0 statistical software was used. A t-test was used for comparison between two groups, where * represents *P*<0.05, ** represents *P*<0.01, and *** represents *P*<0.001. The results are shown in Tables 57-61 and FIGS. 5-9.

Table 57. Inhibitory effects of novel polypeptide HMMW15-51 and micropeptide HMMW on metastasis of human melanoma A375 cells

| Group | Dose (μmol/L) | Number of cell metastases | Inhibition (%) |
|---|---|---|---|
| None | 0 | 590±44.73 | - |
| Novel polypeptide | 50 | 61±30.07 | 89.63*** |
| | 25 | 316±4.57 | 46.40*** |
| HMMW | 50 | 371±13.00 | 37.13*** |

Table 58. Inhibitory effects of novel polypeptide HMMW15-51 and micropeptide HMMW on metastasis of human cervical cancer Hela cells

| Group | Dose (μmol/L) | Number of cell metastases | Inhibition (%) |
|---|---|---|---|
| None | 0 | 1028±121.65 | - |
| Novel polypeptide | 50 | 441±22.66 | 57.13*** |
| | 25 | 448±7.96 | 56.40*** |
| HMMW | 50 | 115+20.32 | 88.80*** |

Table 59. Inhibitory effects of novel polypeptide HMMW15-51 and micropeptide HMMW on metastasis of human ovarian cancer Skvo3 cells

| Group | Dose (μmol/L) | Number of cell metastases | Inhibition (%) |
|---|---|---|---|
| None | 0 | 1138±202.60 | - |
| Novel polypeptide | 50 | 115±21.48 | 89.87*** |
| | 25 | 468±65.96 | 58.90*** |
| HMMW | 50 | 118±26.16 | 89.60*** |

Table 60. Inhibitory effects of novel polypeptide HMMW15-51 and micropeptide HMMW on metastasis of human liver cancer Hep3B cells

| Group | Dose (μmol/L) | Number of cell metastases | Inhibition (%) |
|---|---|---|---|
| None | 0 | 1238±83.12 | - |
| Novel polypeptide | 50 | 50±28.14 | 89.65*** |
| | 25 | 583±75.82 | 48.90*** |
| HMMW | 50 | 70±75.82 | 88.13*** |

Table 61. Inhibitory effects of novel polypeptide HMMW15-51 and micropeptide HMMW on metastasis of human lung cancer A549 cells

| Group | Dose (μmol/L) | Number of cell metastases | Inhibition (%) |
|---|---|---|---|
| None | 0 | 623±29.92 | - |

(continued)

| Group | Dose (μmol/L) | Number of cell metastases | Inhibition (%) |
|---|---|---|---|
| Novel polypeptide | 50 | 64±32.44 | 89.62*** |
| | 25 | 43±19.22 | 92.97*** |
| HMMW | 50 | 33±20.50 | 94.57*** |

According to Tables 57-61 and FIGS. 5-9, compared with the blank control, the novel polypeptide HMMW15-51 has significant inhibitory effects on metastasis of human melanoma, cervical cancer, ovarian cancer, liver cancer, and lung cancer cells, indicating that the novel polypeptide HMMW15-51 may be developed into anti-tumor drugs by inhibiting metastasis of the malignant tumor cells.

**Example 7**

This example provides detection of in vivo effects of the novel polypeptide HMMW15-51 (SEQ ID NO.21) and the micropeptide HMMW (SEQ ID NO.1) on a subcutaneous transplanted tumor model of tongue squamous cell carcinoma cells CAL27.

(1) A large number of human tongue squamous cell carcinoma CAL27 cells were cultured and digested with a 0.25% trypsin solution. After digestion was terminated, a cell suspension was centrifuged at 1000 rpm for 5 min, and the cells were resuspended in a serum-free DMEM culture medium and counted. The cell concentration was adjusted to $5\times10^7$ cells/mL.
(2) BALB/c nude mice (4-6 week old, female, weighing 14-16 g, adaptively fed in an SPF animal feeding room for 1 week) were inoculated with 100 μL of cell suspensions of corresponding groups under the left axilla, with an injection volume of $5\times10^6$ cells/mouse;
(3) After inoculation, the tumor growth at the inoculation sites of the nude mice was closely observed. On the 7$^{th}$ day after inoculation, white nodules appeared at the inoculation sites and could move subcutaneously upon touch. As the tumor tissues grew, hard tumor blocks gradually formed at the inoculation sites, and the average volume of the tumor tissues reached 80 $mm^3$ in about 14 days. The BALB/c nude mice were randomly divided into 3 groups (a normal saline group served as a blank control, and the dose of the micropeptide HMMW and the novel polypeptide HMMW15-51 was 2.7 μM/kg), with 10 mice in the blank group and 6 mice in each other group. At the beginning of administration, the animal weight was about 20 g.
(4) The volume of the transplanted tumor was measured and recorded every three days. The formula for calculating the tumor volume (TV) is as follows:

$$\text{Tumor volume}=0.5\times a\times b^2,$$

where a is the length (mm) of the transplanted tumor and b is the width (mm) of the transplanted tumor.
(5) After drug administration, the nude mice were euthanized, and the tumor blocks were removed and weighed to calculate the tumor growth inhibition by a calculation formula as follows:

$$\text{Tumor growth inhibition}=(1-T/C)\times100\%,$$

where T is the average tumor weight of the treatment group, and C is the average tumor weight of the control group.

Data was presented as mean ± SD, and GraphPad Prism 5.0 statistical software was used. A t-test was used for comparison between two groups, while One-way Anova was used for comparison among a plurality of groups. **** represents $P<0.0001$.
As shown in FIG. 10, compared with the normal saline control group, the micropeptide HMMW and the novel polypeptide HMMW15-51 can significantly inhibit the in vivo tumorigenicity of the human tongue squamous cell carcinoma CAL27 cells to varying degrees, and the activity of the HMMW15-51 is not significantly different from that of the HMMW.

**Example 8**

This example provides detection of inhibitory effects of the novel polypeptide HMMW 15-51 (SEQ ID NO.21)

obtained by long-acting modification on proliferation of different tumor cells.

In this example, the long-acting modification comprises N-terminus linkage with an 18-carbon fatty acid, namely a peptide C18-HMMW15-51 obtained by modification, and linkage with an 18-carbon fatty acid using a small peptide Ala-Ala-Asn as a linker, namely C18-L-HMMW15-51.

The structure of the C18-HMMW15-51 is:

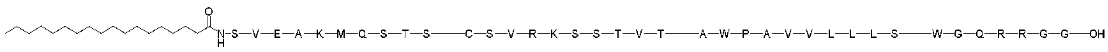

The structure of the C18-L-HMMW15-51 is:

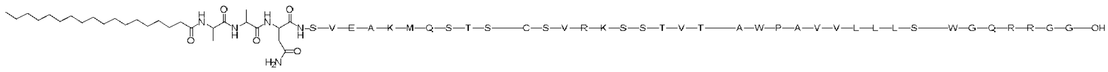

The C18-HMMW15-51 and the C18-L-HMMW15-51 can be coupled with amino acids by solid-phase synthesis. If a fatty acid needs to be coupled, the fatty acid can be coupled to a peptide resin according to an amino acid synthesis scheme (solid-phase synthesis) to obtain a crude product. The crude product is cleaved, subjected to preparative high-performance liquid chromatography, and freeze-dried to obtain a target compound.

When human tongue squamous cell carcinoma cells CAL27, ovarian cancer cells SKOV3, and liver cancer cells Hep3B were cultured to a density of 90% in a 37°C, 5% $CO_2$ incubator, the cells were digested with trypsin and collected, resuspended in a culture solution, and counted under a microscope. The cell concentration was adjusted to approximately $1.0\times10^5$ cells/mL, and a cell suspension was seeded into a 96-well plate with 100 μL per well, and cultured overnight in a 37°C, 5% $CO_2$ incubator. After the cells fully adhered to the wall, 100 μL of the HMMW15-51, C18-HMMW15-51, and C18-L-HMMW15-51 having a concentration of 100 μM were added separately as a treatment group; and a culture solution without any drugs was used as a negative control group. After incubation was continued for 48 h, 10 μL of CCK8 was added per well in a 96-well plate for dissolution. Incubation was continued for 4 h in a cell incubator, an absorbance was measured at 450 nm, and a proliferation inhibition was calculated. Data was presented as mean+SD, and GraphPad Prism 5.0 statistical software was used. A t-test was used for comparison between two groups, while One-way Anova was used for comparison among a plurality of groups.

As shown in FIGS. 11-13, **** represents P<0.0001, and the inhibitory effects of the novel polypeptide HMMW15-51 (SEQ ID NO.21), the peptide C18-HMMW15-51 obtained by modification, and the C18-L-HMMW15-51 obtained by modification on proliferation of different tumor cells have no significant difference.

## Example 9

This example provides detection of in vivo effects of the novel polypeptides HMMW15-51 (SEQ ID NO.21), C18-HMMW15-51, and C18-L-HMMW15-51 on a subcutaneous transplanted tumor model of tongue squamous cell carcinoma cells CAL27.

(1) A large number of human tongue squamous cell carcinoma cells CAL27 were cultured and digested with a 0.25% trypsin solution. After digestion was terminated, a cell suspension was centrifuged at 1000 rpm for 5 min, and the cells were resuspended in a serum-free DMEM culture medium and counted. The cell concentration was adjusted to $5\times10^7$ cells/mL.

(2) BALB/c nude mice (4-6 week old, female, weighing 14-16 g, adaptively fed in an SPF animal feeding room for 1 week) were inoculated with 100 μL of cell suspensions of corresponding groups under the left axilla, with an injection volume of $5\times10^6$ cells/mouse;

(3) After inoculation, the tumor growth at the inoculation sites of the nude mice was closely observed. On the 7th day after inoculation, white nodules appeared at the inoculation sites and could move subcutaneously upon touch. As the tumor tissues grew, hard tumor blocks gradually formed at the inoculation sites, and the average volume of the tumor tissues reached 80 $mm^3$ in about 14 days. The BALB/c nude mice were randomly divided into 4 groups: a normal saline group serving as a blank control, a micropeptide HMMW15-51 group (intravenously injected every day), a C18-HMMW 15-51 group (subcutaneously injected on alternate days), and a C18-L-HMMW15-51 group (subcutaneously injected on alternate days), and the dose was 2.7 μM/kg, with 10 mice in the blank group and 6 mice in each other group. At the beginning of administration, the animal weight was about 20 g.

(4) The volume of the transplanted tumor was measured and recorded every three days. The formula for calculating the tumor volume (TV) is as follows:

$$\text{Tumor volume}=0.5\times a\times b^2,$$

where a is the length (mm) of the transplanted tumor and b is the width (mm) of the transplanted tumor.
(5) After drug administration for 21 days, the nude mice were euthanized, and the tumor blocks were removed and weighed to calculate the tumor growth inhibition by a calculation formula as follows:

$$\text{Tumor growth inhibition}=(1\text{-}T/C)\times 100\%,$$

where T is the average tumor weight of the treatment group, and C is the average tumor weight of the control group.

Data was presented as mean ± SD, and GraphPad Prism 5.0 statistical software was used. A t-test was used for comparison between two groups, while One-way Anova was used for comparison among a plurality of groups.
As shown in FIG. 14, * represents P<0.05, ** represents P<0.01, and *** represents P<0.001, compared with the normal saline control group, the micropeptide HMMW15-51 group (intravenously injected every day), the C18-HMMW15-51 group (subcutaneously injected on alternate days), and the C18-L-HMMW15-51 group (subcutaneously injected on alternate days) can significantly inhibit the in vivo tumorigenicity of the human tongue squamous cell carcinoma cells CAL27 to varying degrees, and compared with the HMMW15-51, the C18-L-HMMW15-51 has better inhibitory effects upon reduction in the number of administration times and optimization of an administration route.

## Claims

1. Use of a novel polypeptide or a derivative thereof in the preparation of a medicament for preventing or treating tumors, wherein the novel polypeptide is selected from any one of the following:

   (1) a polypeptide with the same or substantially the same function formed by deleting 3 consecutive amino acids from a polypeptide having the amino acid sequence set forth in SEQ ID NO.1;
   (2) a polypeptide with the same or substantially the same function formed by deleting 1 to 34 amino acids at N-terminus of a polypeptide having the amino acid sequence set forth in SEQ ID NO.1; and
   (3) a polypeptide with the same or substantially the same function formed by substituting one or more amino acids of a polypeptide having the amino acid sequence set forth in SEQ ID NO.1, or (1), or (2).

2. The use of a novel polypeptide or a derivative thereof in the preparation of a medicament for preventing or treating tumors according to claim 1, wherein

   in said (1), a polypeptide with the same or substantially the same function formed by deleting 3 consecutive amino acids at positions 3N-2, 3N-1, and 3N from a polypeptide having the amino acid sequence set forth in SEQ ID NO.1, wherein N=1, 2, 3, 4, 5, 6, 7, 8, 9, 10, 11, 12, 13, 14, 15, 16, or 17; or
   in said (2), a polypeptide with the same or substantially the same function formed by deleting 4, 9, 14, 19, 24, 29, or 34 amino acids at N-terminus of a polypeptide having the amino acid sequence set forth in SEQ ID NO.1; or
   in said (3), the amino acid at position 2, 3, 8, 9, 10, 11, 12, 13, 17, 19, 20, 21, 22, 25, 27, 28, 30, 31, 32, 40, 41, 42, 43, 44, 45, 48, 49, or 50 in the amino acid sequence set forth in SEQ ID NO.1 is substituted.

3. The use of a novel polypeptide or a derivative thereof in the preparation of a medicament for preventing or treating tumors according to claim 2, wherein the amino acid sequence of the novel polypeptide is shown as any one of SEQ ID NO.2 to SEQ ID NO.53.

4. The use of a novel polypeptide or a derivative thereof in the preparation of a medicament for preventing or treating tumors according to claim 3, wherein the amino acid sequence of the novel polypeptide is shown as any one of SEQ ID NO.2 to SEQ ID NO.4, SEQ ID NO.6 to SEQ ID NO.10, SEQ ID NO.13, SEQ ID NO.15 to SEQ ID NO.17, SEQ ID NO.19 to SEQ ID NO.21, SEQ ID NO.26 to SEQ ID NO.51, and SEQ ID NO.53.

5. The use of a novel polypeptide or a derivative thereof in the preparation of a medicament for preventing or treating tumors according to any one of claims 1-4, wherein the derivative comprises a derivative with the same or substantially the same function of the novel polypeptide obtained by modification.

6. The use of a novel polypeptide or a derivative thereof in the preparation of a medicament for preventing or treating

tumors according to claim 5, wherein the modification comprises one or more of N-terminus modification, C-terminus modification, side-chain modification, amino acid modification, or backbone modification.

7. The use of a novel polypeptide or a derivative thereof in the preparation of a medicament for preventing or treating tumors according to claim 6, wherein the modification comprises one or more long-acting modification of polyethylene glycol modification, fatty acid linkage, fusion with Fc polypeptide, binding with human serum albumin, non-natural amino acid substitution, polypeptide cyclization, or high-end formulations.

8. The use of a novel polypeptide or a derivative thereof in the preparation of a medicament for preventing or treating tumors according to claim 7, wherein the modification comprises fatty acid linkage, wherein the fatty acid linkage comprises N-terminus linkage with a 12- to 20-carbon fatty acid or linkage with a 12- to 20-carbon fatty acid using a linker.

9. The use of a novel polypeptide or a derivative thereof in the preparation of a medicament for preventing or treating tumors according to claim 8, wherein the fatty acid linkage comprises N-terminus linkage with an 18-carbon fatty acid or linkage with an 18-carbon fatty acid using a linker, wherein the linker is a peptide less than 5 amino acids in length.

10. The use of a novel polypeptide or a derivative thereof in the preparation of a medicament for preventing or treating tumors according to any one of claims 1-9, wherein the tumors comprise one or more of glioma, neuroblastoma, head and neck cancer, esophageal cancer, thyroid cancer, lung cancer, liver cancer, kidney cancer, breast cancer, cervical cancer, uterine cancer, ovarian cancer, colon cancer, small intestine cancer, ileocecal cancer, gastric cancer, bladder cancer, pancreatic cancer, prostate cancer, cholangiocarcinoma, melanoma, sarcoma, myeloma, lymphoma, and leukemia.

11. The use of a novel polypeptide or a derivative thereof in the preparation of a medicament for preventing or treating tumors according to claim 10, wherein the preventing or treating tumors comprises inhibition of proliferation and/or metastasis of tumor cells.

12. The use of a novel polypeptide or a derivative thereof in the preparation of a medicament for preventing or treating tumors according to claim 11, wherein the preventing or treating tumors comprises inhibition of proliferation of tumor cells, wherein the tumors and cells comprise:

wherein the cancer cells of the glioblastoma comprises: U87-MG;
wherein the cancer cells of the neuroblastoma comprises: SH-SY5Y;
wherein the cancer cells of the head and neck cancer comprises: any one of oral epidermoid carcinoma cell KB, laryngeal epidermoid carcinoma cell HEp2, tongue squamous cell carcinoma cell CAL27, and nasopharyngeal carcinoma cell CNE-2Z;
wherein the cancer cells of the esophageal cancer comprises: Eca-109 and/or TE-13;
wherein the cancer cells of the thyroid cancer comprises: NPPA87-1;
wherein the cancer cells of the lung cancer comprises: any one or combination of 95-D, SK-MES-1, SPC-A1, and A549;
wherein the cancer cells of the liver cancer comprises: HCCLM3 and/or HepG2;
wherein the cancer cells of the kidney cancer comprises: Ketr-3 and/or 786-O;
wherein the cancer cells of the breast cancer comprises: SK-BR-3 and/or MDA-MB-231;
wherein the cancer cells of the cervical cancer comprises: CaSki and/or Hela;
wherein the cancer cells of the uterine cancer comprises: RL-952 and/or MS751;
wherein the cancer cells of the ovarian cancer comprises: ES-2 and/or SKOV3;
wherein the cancer cells of the colon cancer comprises: HT29 and/or SW480;
wherein the cancer cells of the small intestine cancer comprises: HIC;
wherein the cancer cells of the ileocecal cancer comprises: HCT-8;
wherein the cancer cells of the gastric cancer comprises: MGC-803 and/or BGC-823;
wherein the cancer cells of the bladder cancer comprises: T24 and/or EJ;
wherein the cancer cells of the pancreatic cancer comprises: ASPC-1 and/or PANC-1;
wherein the cancer cells of the prostate cancer comprises: 22RV1 and/or PC-3;
wherein the cancer cells of the cholangiocarcinoma comprises: QBC939;
wherein the cancer cells of the melanoma comprises: SK-mel-2 and/or A375;
wherein the cancer cells of the sarcoma comprises: MG63 and/or HT-1080;
wherein the cancer cells of the myeloma comprises: LP-1;

wherein the cancer cells of the lymphoma comprises: any one or combination of Raji, RPMI 8226, U937, and WSU-DLCL2; and
wherein the cancer cells of the leukemia comprises: any one or combination of CEM/C1, NB4, KG-1, K562, HL60, Jurkat, and Clone E6-1.

**13.** The use of a novel polypeptide or a derivative thereof in the preparation of a medicament for preventing or treating tumors according to claim 11, wherein the preventing or treating tumors comprises inhibiting metastasis of tumor cells, wherein the tumors and cells comprise:

wherein the cancer cells of the melanoma comprises: A375;
wherein the cancer cells of the cervical cancer comprises: Hela;
wherein the cancer cells of the ovarian cancer comprises: Skvo3;
wherein the cancer cells of the liver cancer comprises: Hep3B; and
wherein the cancer cells of the lung cancer comprises: A549.

**14.** A pharmaceutical composition for preventing or treating tumors, wherein the pharmaceutical composition comprises at least the novel polypeptide or the derivative thereof according to any one of claims 1-9, and a pharmaceutically acceptable carrier, wherein the tumors comprise one or more of glioma, neuroblastoma, head and neck cancer, esophageal cancer, thyroid cancer, lung cancer, liver cancer, kidney cancer, breast cancer, cervical cancer, uterine cancer, ovarian cancer, colon cancer, small intestine cancer, ileocecal cancer, gastric cancer, bladder cancer, pancreatic cancer, prostate cancer, cholangiocarcinoma, melanoma, sarcoma, myeloma, lymphoma, and leukemia.

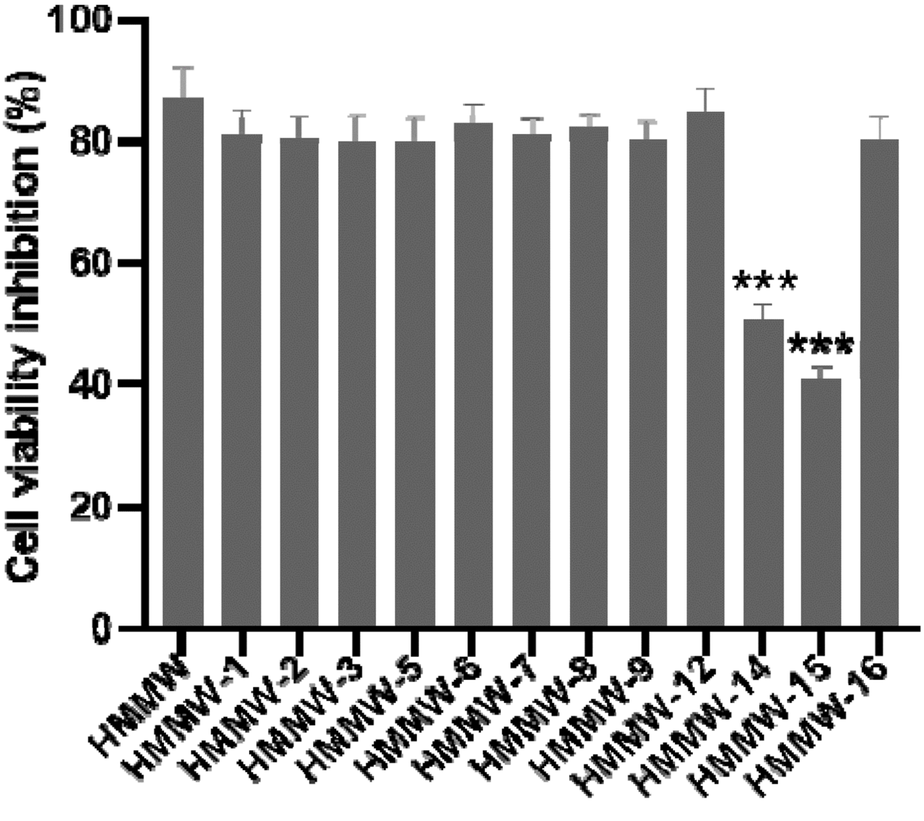
Cell viability inhibition (%)
100
80
60
40
20
0
***
***
HMMW
HMMW-1
HMMW-2
HMMW-3
HMMW-5
HMMW-6
HMMW-7
HMMW-8
HMMW-9
HMMW-12
HMMW-14
HMMW-15
HMMW-16

FIG. 1

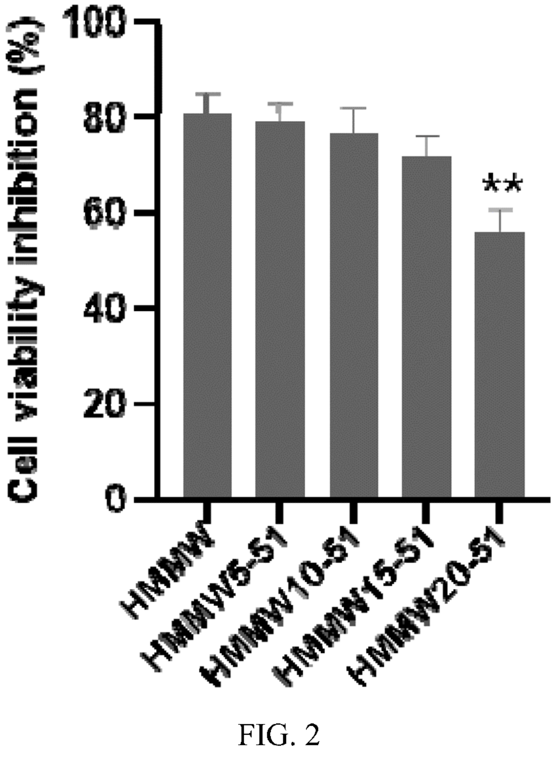
Cell viability inhibition (%)
100
80
60
40
20
0
**
HMMW
HMMW5-51
HMMW10-51
HMMW15-51
HMMW20-51

FIG. 2

| Polypeptide name | Amino acid sequence |
|---|---|
| HMMW | MERAGVPGFSPRRSSVEAKMQSTSCSVRKSSTVTAWPAVVLLLSWGQRRGG |
| HMMW-2A | MARAGVPGFSPRRSSVEAKMQSTSCSVRKSSTVTAWPAVVLLLSWGQRRGG |
| HMMW-3A | MEAAGVPGFSPRRSSVEAKMQSTSCSVRKSSTVTAWPAVVLLLSWGQRRGG |
| HMMW-8A | MERAGVPAFSPRRSSVEAKMQSTSCSVRKSSTVTAWPAVVLLLSWGQRRGG |
| HMMW-9A | MERAGVPGASPRRSSVEAKMQSTSCSVRKSSTVTAWPAVVLLLSWGQRRGG |
| HMMW-10A | MERAGVPGFAPRRSSVEAKMQSTSCSVRKSSTVTAWPAVVLLLSWGQRRGG |
| HMMW-11A | MERAGVPGFSARRSSVEAKMQSTSCSVRKSSTVTAWPAVVLLLSWGQRRGG |
| HMMW-12A | MERAGVPGFSPARSSVEAKMQSTSCSVRKSSTVTAWPAVVLLLSWGQRRGG |
| HMMW-13A | MERAGVPGFSPRASSVEAKMQSTSCSVRKSSTVTAWPAVVLLLSWGQRRGG |
| HMMW-17A | MERAGVPGFSPRRSSVAAKMQSTSCSVRKSSTVTAWPAVVLLLSWGQRRGG |
| HMMW-19A | MERAGVPGFSPRRSSVEAAMQSTSCSVRKSSTVTAWPAVVLLLSWGQRRGG |
| HMMW-20A | MERAGVPGFSPRRSSVEAKAQSTSCSVRKSSTVTAWPAVVLLLSWGQRRGG |
| HMMW-21A | MERAGVPGFSPRRSSVEAKMASTSCSVRKSSTVTAWPAVVLLLSWGQRRGG |
| HMMW-22A | MERAGVPGFSPRRSSVEAKMQATSCSVRKSSTVTAWPAVVLLLSWGQRRGG |
| HMMW-25A | MERAGVPGFSPRRSSVEAKMQSTSASVRKSSTVTAWPAVVLLLSWGQRRGG |
| HMMW-27A | MERAGVPGFSPRRSSVEAKMQSTSCSARKSSTVTAWPAVVLLLSWGQRRGG |
| HMMW-28A | MERAGVPGFSPRRSSVEAKMQSTSCSVAKSSTVTAWPAVVLLLSWGQRRGG |
| HMMW-30A | MERAGVPGFSPRRSSVEAKMQSTSCSVRKASTVTAWPAVVLLLSWGQRRGG |
| HMMW-31A | MERAGVPGFSPRRSSVEAKMQSTSCSVRKSATVTAWPAVVLLLSWGQRRGG |
| HMMW-32A | MERAGVPGFSPRRSSVEAKMQSTSCSVRKSSAVTAWPAVVLLLSWGQRRGG |
| HMMW-40A | MERAGVPGFSPRRSSVEAKMQSTSCSVRKSSTVTAWPAVALLLSWGQRRGG |
| HMMW-41A | MERAGVPGFSPRRSSVEAKMQSTSCSVRKSSTVTAWPAVVALLSWGQRRGG |
| HMMW-42A | MERAGVPGFSPRRSSVEAKMQSTSCSVRKSSTVTAWPAVVLALSWGQRRGG |
| HMMW-43A | MERAGVPGFSPRRSSVEAKMQSTSCSVRKSSTVTAWPAVVLLASWGQRRGG |
| HMMW-44A | MERAGVPGFSPRRSSVEAKMQSTSCSVRKSSTVTAWPAVVLLLAWGQRRGG |
| HMMW-45A | MERAGVPGFSPRRSSVEAKMQSTSCSVRKSSTVTAWPAVVLLLSAGQRRGG |
| HMMW-48A | MERAGVPGFSPRRSSVEAKMQSTSCSVRKSSTVTAWPAVVLLLSWGQARGG |
| HMMW-49A | MERAGVPGFSPRRSSVEAKMQSTSCSVRKSSTVTAWPAVVLLLSWGQRAGG |
| HMMW-50A | MERAGVPGFSPRRSSVEAKMQSTSCSVRKSSTVTAWPAVVLLLSWGQRRAG |

FIG. 3

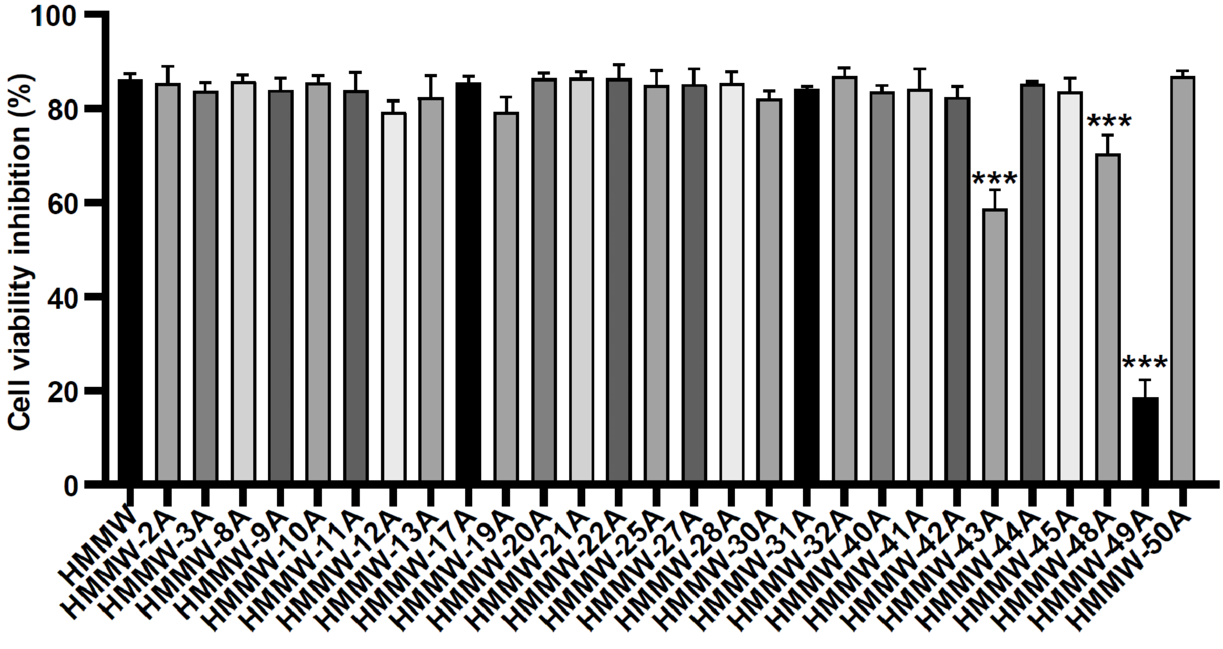
Cell viability inhibition (%)
100
80
60
40
20
0
***
***
***
HMMW
HMMW-2A
HMMW-3A
HMMW-8A
HMMW-9A
HMMW-10A
HMMW-11A
HMMW-12A
HMMW-13A
HMMW-17A
HMMW-19A
HMMW-20A
HMMW-21A
HMMW-22A
HMMW-25A
HMMW-27A
HMMW-28A
HMMW-30A
HMMW-31A
HMMW-32A
HMMW-40A
HMMW-41A
HMMW-42A
HMMW-43A
HMMW-44A
HMMW-45A
HMMW-48A
HMMW-49A
HMMW-50A

FIG. 4

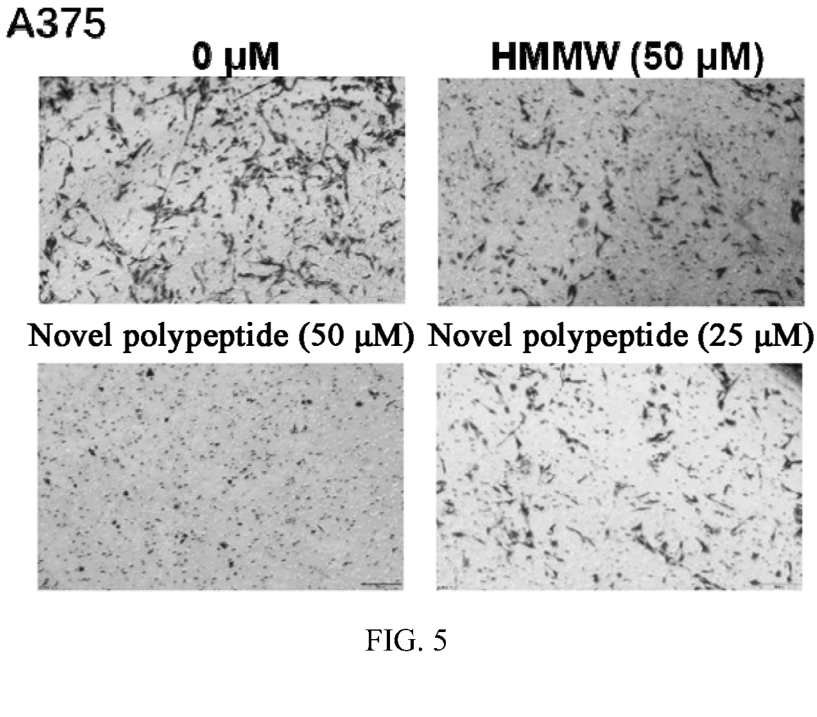
A375
0 μM
HMMW (50 μM)
Novel polypeptide (50 μM)
Novel polypeptide (25 μM)

FIG. 5

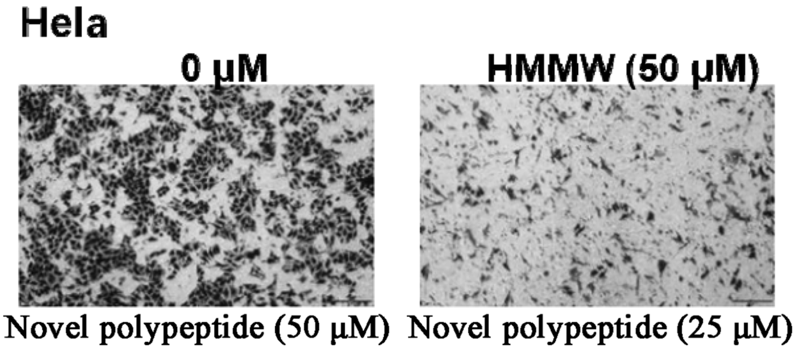


FIG. 6

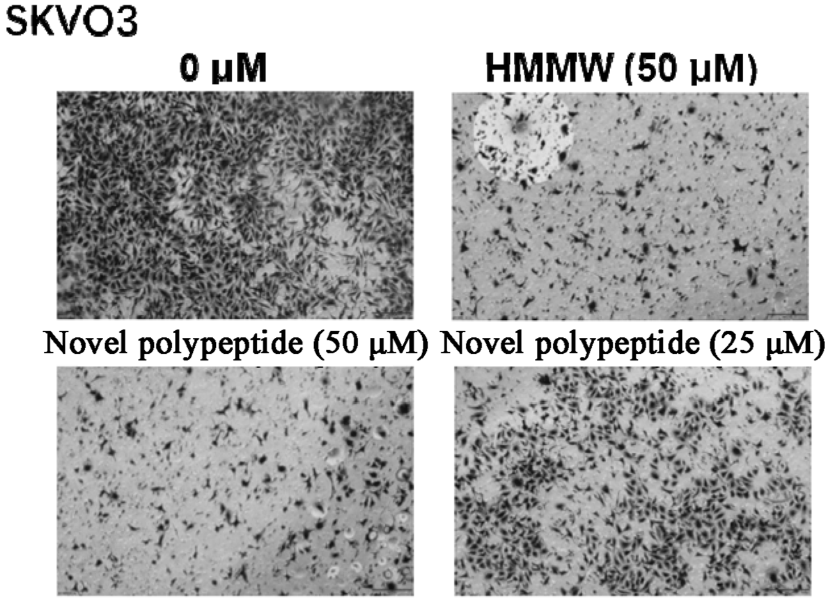


FIG. 7

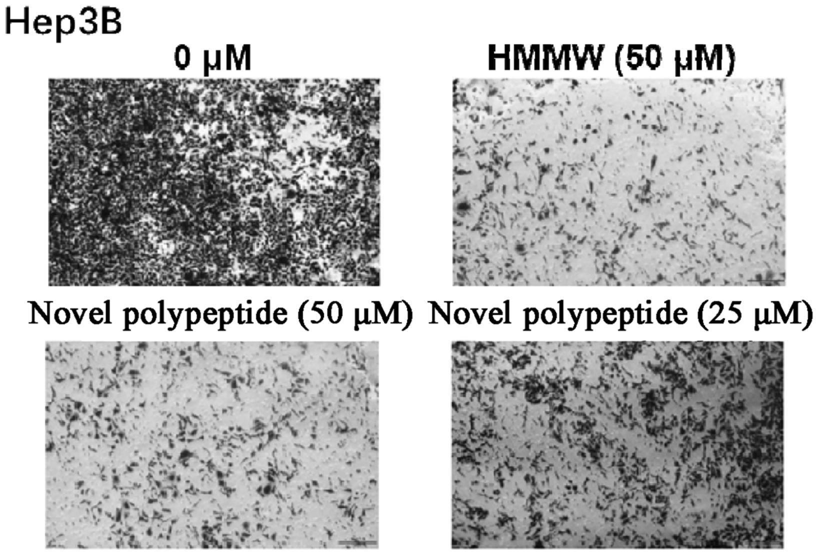


FIG. 8

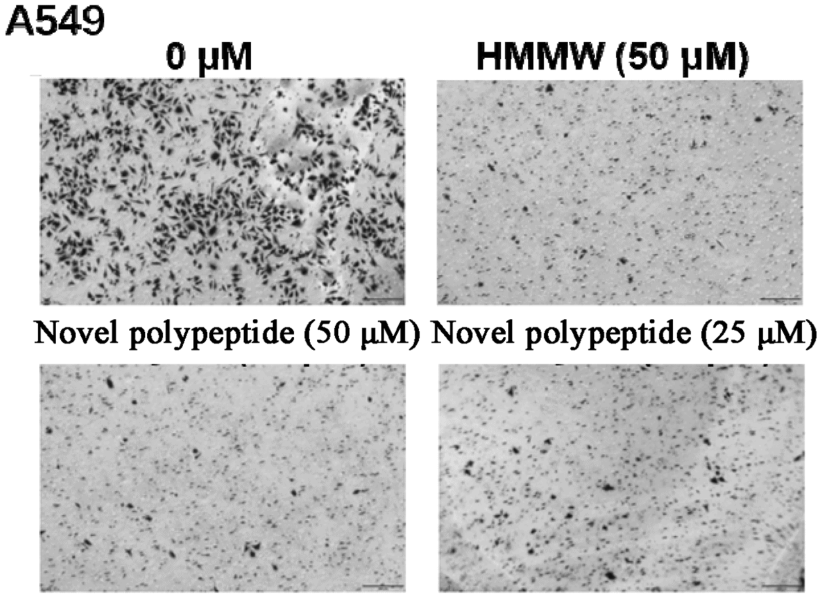
A549
0 μM
HMMW (50 μM)
Novel polypeptide (50 μM)
Novel polypeptide (25 μM)

FIG. 9

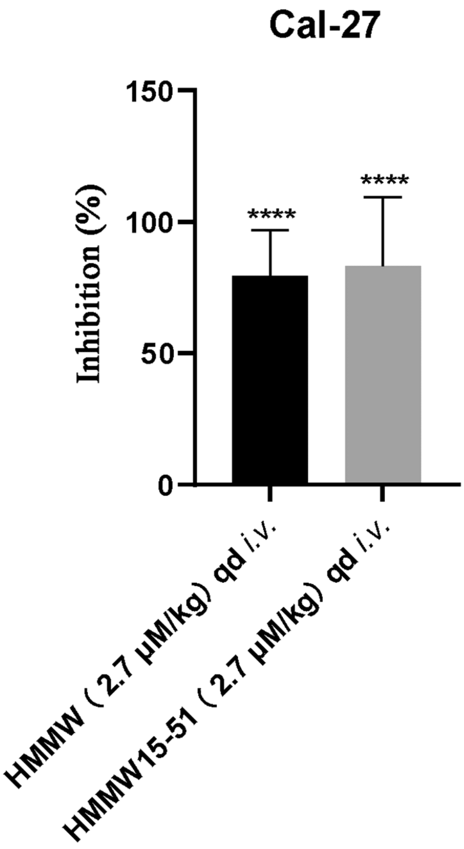
Cal-27
150
100
50
0
Inhibition (%)
****
****
HMMW（2.7 μM/kg) qd i.v.
HMMW15-51（2.7 μM/kg) qd i.v.

FIG. 10

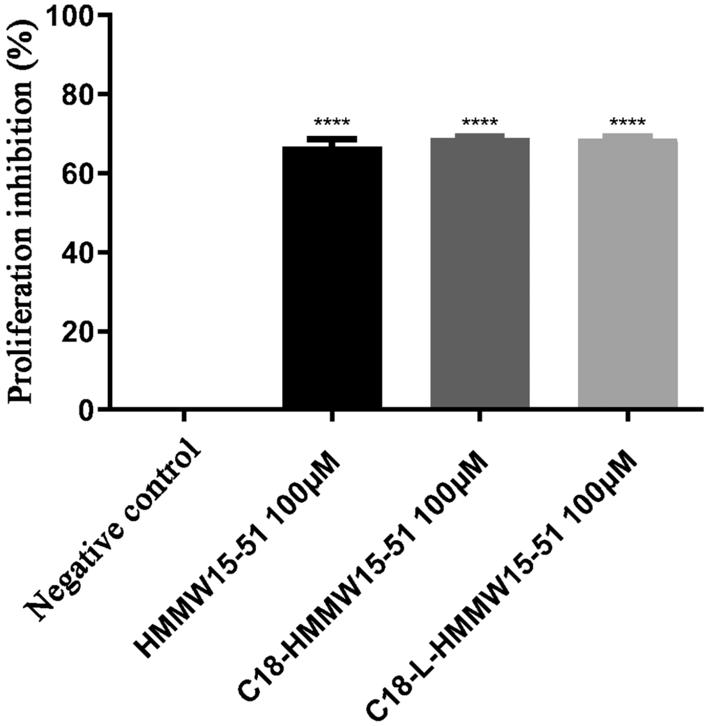
Proliferation inhibition (%)
100
80
60
40
20
0
****
****
****
Negative control
HMMW15-51 100μM
C18-HMMW15-51 100μM
C18-L-HMMW15-51 100μM

FIG. 11

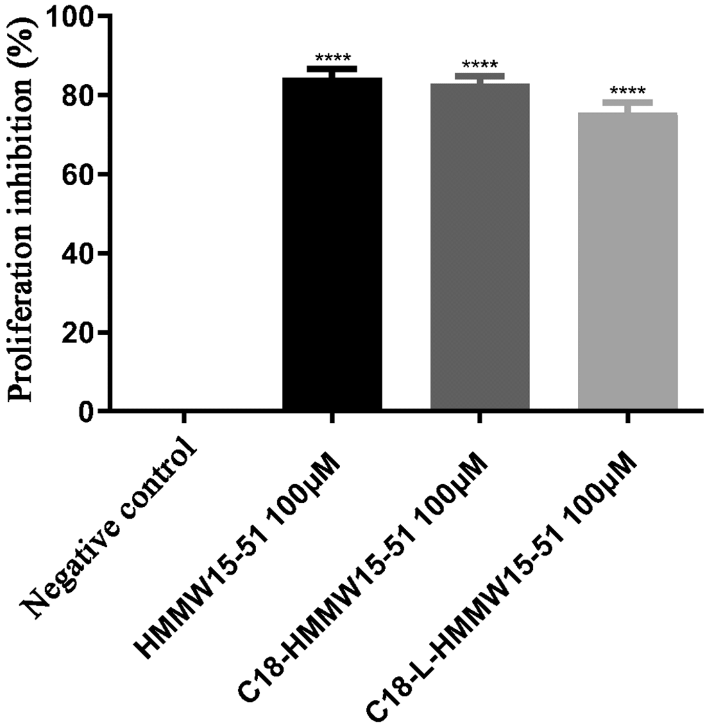
Proliferation inhibition (%)
100
80
60
40
20
0
****
****
****
Negative control
HMMW15-51 100μM
C18-HMMW15-51 100μM
C18-L-HMMW15-51 100μM

FIG. 12

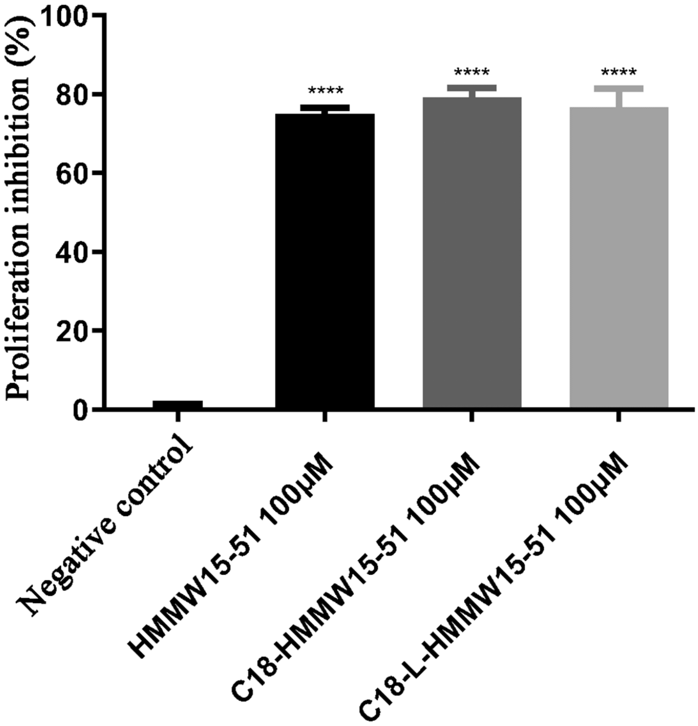
Proliferation inhibition (%)
100
80
60
40
20
0
****
****
****
Negative control
HMMW15-51 100μM
C18-HMMW15-51 100μM
C18-L-HMMW15-51 100μM

FIG. 13

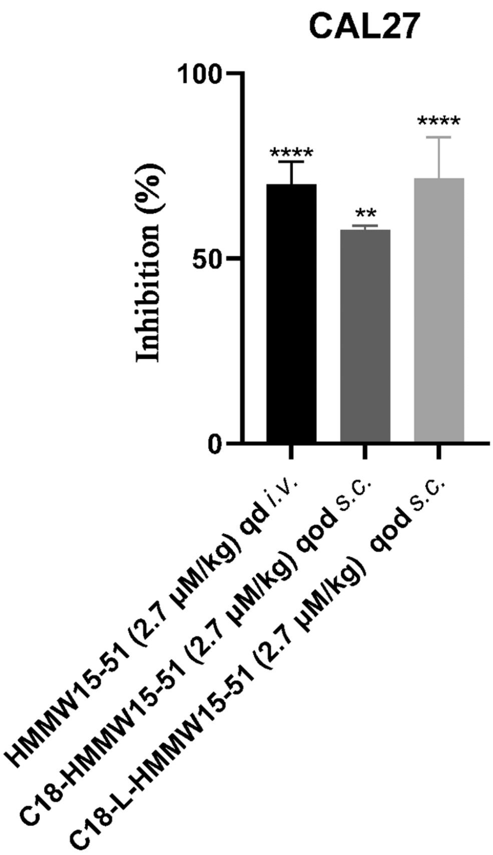
CAL27
Inhibition (%)
100
50
0
****
**
****
HMMW15-51 (2.7 μM/kg) qd i.v.
C18-HMMW15-51 (2.7 μM/kg) qod s.c.
C18-L-HMMW15-51 (2.7 μM/kg) qod s.c.

FIG. 14

**INTERNATIONAL SEARCH REPORT**

International application No. **PCT/CN2024/121856**

**A. CLASSIFICATION OF SUBJECT MATTER**

A61K 38/17(2006.01)i; C07K 14/47(2006.01)i; C12N 15/12(2006.01)i; C12Q 1/6886(2018.01)i; C12N 15/85(2006.01)i; A61K 38/16(2006.01)i; A61P 35/00(2006.01)i; G01N 33/68(2006.01)i; G01N 33/574(2006.01)i

According to International Patent Classification (IPC) or to both national classification and IPC

**B. FIELDS SEARCHED**

Minimum documentation searched (classification system followed by classification symbols)

IPC:A61K C07K C12N C12Q A61P G01N

Documentation searched other than minimum documentation to the extent that such documents are included in the fields searched

Electronic data base consulted during the international search (name of data base and, where practicable, search terms used)

CNTXT, ENTXT, ENTXTC, VEN, CNKI, NCBI, 万方数据库, WANFANG DATABASE, pubmed, ISI web of science, stn: 胞: ncRNA, 非编码RNA, 微肽, aqp2, 缺失, 截短, 截断, N端, 突变, delet+, mutat+, truncat+, segment, HMMW, miac, sORF, non-coding rna, aqp-2, micropeptide?, micropeptide inhibiting cytoskeleton, lncRNAAC025514.2, endogenous peptide, SEQ ID NO: 1-53

**C. DOCUMENTS CONSIDERED TO BE RELEVANT**

| Category* | Citation of document, with indication, where appropriate, of the relevant passages | Relevant to claim No. |
|---|---|---|
| PX | LI, Mengwei et al. "RecName: Full=Micropeptide inhibiting actin cytoskeleton" *UniProtKB/Swiss-Prot: A0A7L8Y648.1,* 24 January 2024 (2024-01-24), section "Features" | 1-4 |
| E | CN 118957059 A (NANJING ANJI BIOTECHNOLOGY CO., LTD.) 15 November 2024 (2024-11-15) claims 1-10 | 1-14 |
| Y | CN 112442116 A (NANJING ANJI BIOTECHNOLOGY CO., LTD.) 05 March 2021 (2021-03-05) description, paragraphs 0014-0038 | 1-14 |
| Y | LI, Mengwei et al. "Micropeptide MIAC Inhibits HNSCC Progression by Interacting with Aquaporin 2" *Journal of the American Chemical Society,* Vol. 142, 16 March 2020 (2020-03-16), abstract, figure 4, page 6712, right column, last paragraph to page 6713, left column, paragraph 1, and page 6713, right column, paragraphs 1-2 | 1-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 November 2024** | **30 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/121856**

**C. DOCUMENTS CONSIDERED TO BE RELEVANT**

| Category* | Citation of document, with indication, where appropriate, of the relevant passages | Relevant to claim No. |
|---|---|---|
| A | CN 114014928 A (NANJING ANJI BIOTECHNOLOGY CO., LTD.) 08 February 2022 (2022-02-08)<br>claims 1-10 | 1-14 |
| A | CN 116196409 A (NANJING ANJI BIOTECHNOLOGY CO., LTD.) 02 June 2023 (2023-06-02)<br>claims 1-7 | 1-14 |
| A | LI, Mengwei et al. "Micropeptide MIAC Inhibits the Tumor Progression by Interacting with AQP2 and Inhibiting EREG/EGFR Signaling in Renal Cell Carcinoma"<br>*Molecular Cancer,* Vol. 21, 19 September 2022 (2022-09-19),<br>abstract | 1-14 |
| A | SETRERRAHMANE, S. et al. "Cancer-Related Micropeptides Encoded by ncRNAs: Promising Drug Targets and Prognostic Biomarkers"<br>*Cancer Letters,* Vol. 547, 07 May 2022 (2022-05-07),<br>abstract | 1-14 |
| A | POLYCARPOU-SCHWARZ, M. et al. "The Cancer-Associated Microprotein CASIMO1 Controls Cell Proliferation and Interacts with Squalene Epoxidase Modulating Lipid Droplet Formation"<br>*Oncogene,* 16 May 2018 (2018-05-16),<br>abstract | 1-14 |

Form PCT/ISA/210 (second sheet) (July 2022)

**INTERNATIONAL SEARCH REPORT**

International application No.
**PCT/CN2024/121856**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/121856**

| Patent document cited in search report | | | Publication date (day/month/year) | Patent family member(s) | Publication date (day/month/year) |
|---|---|---|---|---|---|
| CN | 118957059 | A | 15 November 2024 | None | |
| CN | 112442116 | A | 05 March 2021 | None | |
| CN | 114014928 | A | 08 February 2022 | None | |
| CN | 116196409 | A | 02 June 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 112442116 A
- CN 112442116 B